# EUROPEAN PATENT SPECIFICATION

(11) **EP 3 443 115 B1**
(45) Date of publication and mention of the grant of the patent: **09.10.2024**
(21) Application number: 17718883.6
(22) Date of filing: 14.04.2017
(51) Int. Cl.: C12Q 1/68

(54) **METHOD FOR THE GENERATION OF DNA LIBRARIES FOR MASSIVE PARALLEL SEQUENCING**
VERFAHREN ZUR ERZEUGUNG VON DNA-BIBLIOTHEKEN FÜR MASSIVE PARALLELE SEQUENZIERUNG
PROCÉDÉ POUR LA GÉNÉRATION DE BANQUES D'ADN POUR UN SÉQUENÇAGE MASSIF PARALLÈLE

(30) Priority: 15.04.2016 IT UA20162640
(43) Date of publication of application: 20.02.2019
(73) Proprietor: Menarini Silicon Biosystems S.p.A., 40013 Castel Maggiore (IT)
(72) Inventor: MANARESI, Nicolò, 40125 Bologna (IT); BUSON, Genny, 40013 Castel Maggiore (IT); TONONI, Paola, 40013 Castel Maggiore (IT)
(74) Representative: Studio Torta S.p.A.
(86) International application number: PCT/EP2017/059075
(87) International publication number: WO 2017/178655

(56) References cited:
- WO-A1-00/17390
- WO-A1-2007/073165
- WO-A1-2014/039556
- WO-A1-2014/071361
- WO-A1-2014/088979
- WO-A1-2015/148219
- WO-A1-2015/171656
- WO-A2-2009/116863
- US-A1- 2014 256 571
- CASSANDRA L HODGKINSON ET AL: "Tumorigenicity and genetic profiling of circulating tumor cells in small-cell lung cancer", NATURE MEDICINE, vol. 20, no. 8, 1 June 2014 (2014-06-01), New York, pages 897 - 903, XP055400376, ISSN: 1078-8956, DOI: 10.1038/nm.3600
- VERA BINDER ET AL: "A New Workflow for Whole-Genome Sequencing of Single Human Cells", HUMAN MUTATION, JOHN WILEY & SONS, INC, US, vol. 35, no. 10, 18 August 2014 (2014-08-18), pages 1260 - 1270, XP071975340, ISSN: 1059-7794, DOI: 10.1002/HUMU.22625
- HOU YONG ET AL: "Comparison of variations detection between whole-genome amplification methods used in single-cell resequencing", GIGASCIENCE, vol. 4, no. 1, 6 August 2015 (2015-08-06), XP055775798, Retrieved from the Internet <URL:http://link.springer.com/article/10.1186/s13742-015-0068-3/fulltext.html> DOI: 10.1186/s13742-015-0068-3
- MICHAEL BAYM ET AL: "Inexpensive Multiplexed Library Preparation for Megabase-Sized Genomes", PLOS ONE, vol. 10, no. 5, 22 May 2015 (2015-05-22), pages e0128036, XP055322764, DOI: 10.1371/journal.pone.0128036
- SYLVIE SANSCHAGRIN ET AL: "Next-generation Sequencing of 16S Ribosomal RNA Gene Amplicons", JOURNAL OF VISUALIZED EXPERIMENTS, no. 90, 29 August 2014 (2014-08-29), XP055253351, DOI: 10.3791/51709

## Description

### Technical Field of the Invention

The present invention relates to a method for low-pass whole-genome sequencing and genome-wide copy-number profiling.

### Prior Art

With single cells it is useful to carry out a Whole Genome Amplification (WGA) for obtaining more DNA in order to simplify and/or make it possible to carry out different types of genetic analyses, including sequencing, SNP detection etc.

WGA with a LM-PCR based on a Deterministic Restriction Site (as described in e.g. WO/2000/017390) is known from the art (herein below referred to simply as DRS-WGA). DRS-WGA has been demonstrated to be a better solution for the amplification of single cells (Ref: Lee YS, et al: Comparison of whole genome amplification methods for further quantitative analysis with microarray-based comparative genomic hybridization. Taiwan J Obstet Gynecol. 2008, 47(1):32-41.) and also more resilient to DNA degradation due to fixing (ref. Stoecklein N.H. et al: SCOMP is Superior to Degenerated Oligonucleotide Primed-PCR for Global Amplification of Minute Amounts of DNA from Microdissected Archival Samples. American Journal of Pathology 2002, Vol. 161, No. 1).

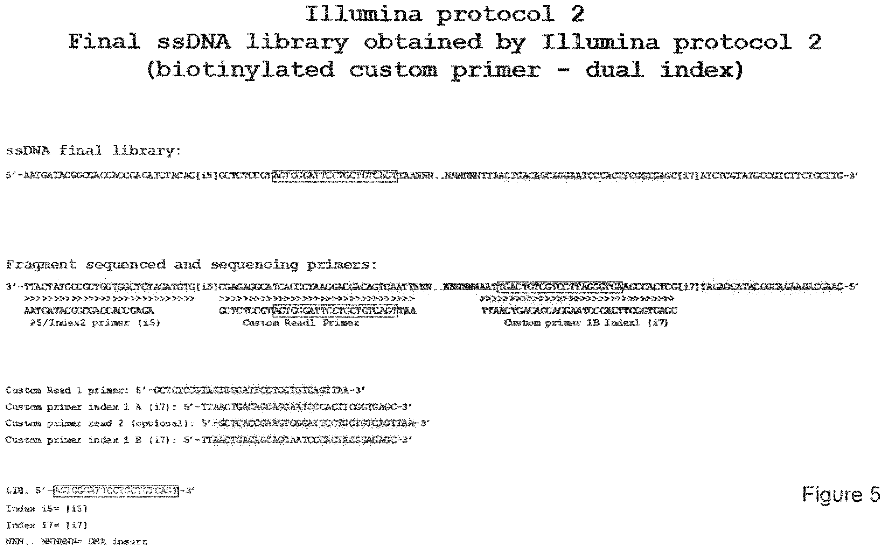

A LM-PCR based, DRS-WGA commercial kit (*Ampli*1*^{™}* WGA kit, Silicon Biosystems) has been used in Hodgkinson C.L. et al., Tumorigenicity and genetic profiling of circulating tumor cells in small-cell lung cancer, Nature Medicine 20, 897-903 (2014). In this work, a Copy-Number Analysis by low-pass whole genome sequencing on single-cell WGA material was performed. However, for the standard workflow used in this paper, the creation of Illumina libraries required several steps, which included i) digestion of WGA adaptors, ii) DNA fragmentation, and standard Illumina workflow steps such as iii) EndRepair iv) A-Tailing v) barcoded adaptor ligation, plus the usual steps of vi) sample pooling of barcoded NGS libraries and vii) sequencing. As shown in the aforementioned article (Fig 5b), WBC did present few presumably falsepositive copy-number calls, although CTCs in general displayed many more aberrations.

*Ampli*1*^{™}* WGA is compatible with array Comparative Genomic Hybridization (aCGH); indeed several groups (Moehlendick B, et al. (2013) A Robust Method to Analyze Copy Number Alterations of Less than 100 kb in Single Cells Using Oligonucleotide Array CGH. PLoS ONE 8(6): e67031; Czyz ZT, et al (2014) Reliable Single Cell Array CGH for Clinical Samples. PLoS ONE 9(1): e85907) showed that it is suitable for high-resolution copy number analysis. However, aCGH technique is expensive and labor intensive, so that different methods such as low-pass whole-genome sequencing (LPWGS) for detection of somatic Copy-Number Alterations (CNA) may be desirable.

Baslan et al (Optimizing sparse sequencing of single cells for highly multiplex copy number profiling, Genome Research, 25:1-11, April 9, 2015), achieved whole-genome copy-number profiling starting from DOP-PCR whole-genome amplification, using several enzymatic steps, including WGA adaptor digestion, ligation of Illumina adapters, PCR amplification.

Yan et al. Proc Natl Acad Sci U S A. 2015 Dec 29; 112 (52) :15964-9, teaches the use of MALBAC WGA (Yikon Genomics Inc), for pre-implantation genetic diagnosis simultaneous for chromosome abnormalities and monogenic disease.

US8206913B1 (Kamberov et al, Rubicon Genomics) teaches an approach where a special Degenerate-Oligonucleotide-Priming-PCR (DOP-PCR), is adopted. This reference also contains an overview of different WGA methods and state of the art. US8206913B1 is at the base of the commercial kit PicoPlex.

Hou et al., Comparison of variations detection between whole-genome amplification methods used in single-cell resequencing, GigaScience (2015) 4:37, reports a performance comparison of several WGA methods, including MALBAC and Multiple Displacement Amplification (MDA). LPWGS and WGS are used in the paper. Library preparation is obtained with workflows

DRS-WGA has been shown to be better than DOP-PCR for the analysis of copy-number profiles from minute amounts of microdissected FFPE material (Stoecklein et al., SCOMP is superior to degenerated oligonucleotide primed-polymerase chain reaction for global amplification of minute amounts of DNA from microdissected archival tissue samples, Am J Pathol. 2002 Jul;161(1):43-51; Arneson et al., Comparison of whole genome amplification methods for analysis of DNA extracted from microdissected early breast lesions in formalin-fixed paraffin-embedded tissue, ISRN Oncol. 2012;2012:710692. doi: 10.5402/2012/710692. Epub 2012 Mar 14.), when using array CGH (Comparative Genome Hybridization), metaphase CGH, as well as for other genetic analysis assay such as Loss of heterozygosity.

WO2014068519 (Fontana et al.) teaches a method for detecting mutations from DRS-WGA products in loci where the mutation introduces, removes or alters a restriction site.

WO2015083121A1 (Klein et al.) teaches a method to assess the genome integrity of a cell and/or the quality of a DRS-WGA product by a multiplex PCR, as further detailed and reported in Polzer et al. EMBO Mol Med. 2014 Oct 30; 6 (11) :1371-86.

Although the DRS-WGA provides best results in terms of uniform and balanced amplification, current protocols based on aCGH or metaphase CGH are laborious and/or expensive. Low-pass whole-genome sequencing has been proposed as a high-throughput method to analyse several samples with higher processivity and lower cost than aCGH. However, known methods for the generation of a massively parallel sequencing library for WGA products (such as DRS-WGA) still require protocols including several enzymatic steps and reactions.

Beyond the application to CTC analysis cited above, also for other single-cell analysis applications, such as prenatal diagnosis on blastocysts, as well as for circulating fetal cells harvested from maternal blood, it would be desirable to have a more streamlined method, combining the reproducibility and quality of DRS-WGA with the capability to analyse genome-wide Copy-Number Variants (CNVs). In addition, determining a whole-genome copy number profile also from minute amount of cells, FFPE or tissue biopsies would be desirable.

WO 2014/071361 discloses a method of preparing a library for sequencing comprising adding stem loop adaptor oligos to fragmented genomic DNA. The loops are then cleaved resulting in genome fragments flanked by double stranded adaptors. The fragments are then amplified with primers comprising a barcode and used for DNA sequencing on a Ion Torrent sequencing platform.

This method has a series of drawbacks, the most important of which are:
- the method involves a number of subsequent steps involving several reactions and several enzymes;
- the method is not applicable as such on DNA deriving from a single-cell sample.

### Summary of the Invention

One object of the present invention is to provide a method for low-pass whole genome sequencing which is simpler and requires less steps with respect to prior art methods . In particular it is an object of the present invention to provide a method that includes less enzymatic reactions than generally reported in the literature.

Another object of the invention is to provide a method to generate a genome-wide copy-number profile using the method for low-pass whole genome sequencing according to the invention.

Preferably the library used in the low-pass whole genome sequencing method should be compatible with a selected sequencing platform, e.g. Ion Torrent-platform or Illuminaplatform.

The present invention relates to a method to generate a massively parallel sequencing library for Whole Genome Sequencing from Whole Genome Amplification products as defined in the appended claims. The invention further relates to a method to generate a genome-wide copy-number profile using the method for low-pass whole genome sequencing of the invention.

Primer sequences and operative protocols are also provided.

Preferably, the library generation reaction comprises the introduction of a sequencing barcode for multiplexing several samples in the same NGS run. Preferably, the WGA is a DRS-WGA and the library is generated with a single-tube, one-step PCR reaction.

### Brief Description of the Drawings

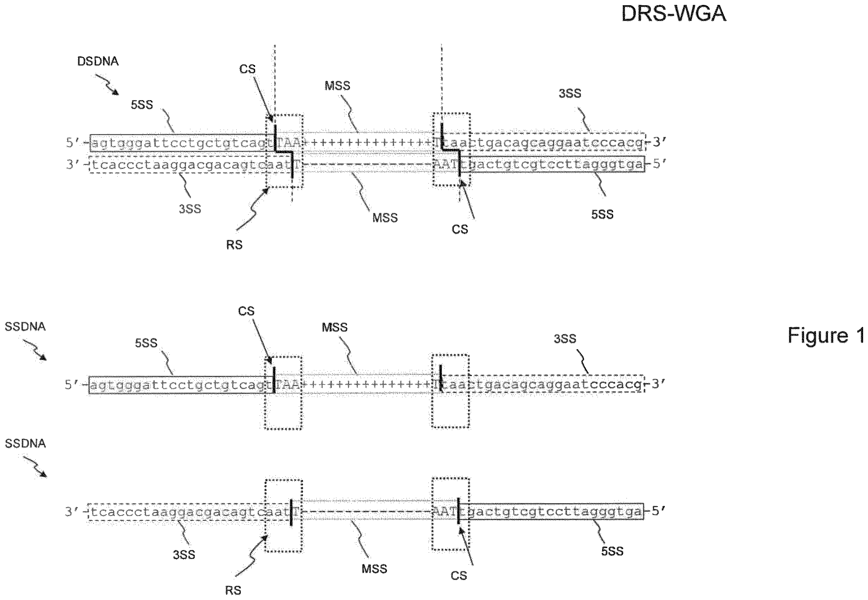

Figure 1 shows a starting product to be used in a first embodiment of the invention, consisting in a DRS-WGA generated DNA library, of which a single fragment is illustrated in a purely schematic way;

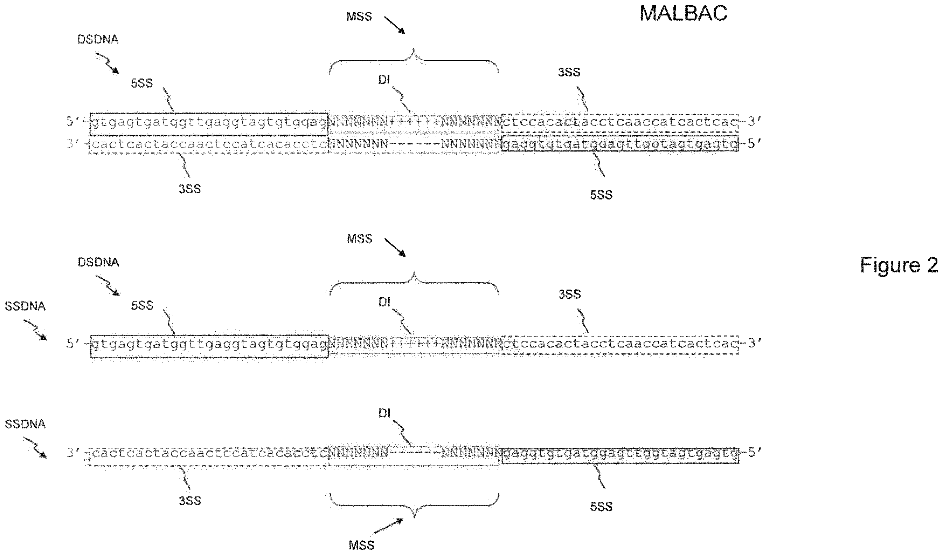

Figure 2 shows a starting product to be used in a second embodiment of the invention, consisting in a MALBAC generated DNA library, of which a single fragment is illustrated in a purely schematic way;
Figure 3 shows in a schematic way an embodiment of the re-amplification step of the method of generating a massively parallel sequencing library disclosed herein applied to the fragment of a DRS-WGA generated DNA library as shown in figure 1 and directed to provide a DNA library compatible with a sequencing platform of the kind of the Ion Torrent or Illumina sequencing platform;
Figure 4 shows in a schematic way the protocol workflow that includes a re-amplification reaction step applied to the fragment of a DRS-WGA as shown in figure 1, and subsequently a fragment library selection. This method provides directly a DNA library compatible with the ILLUMINA sequencing platform;
Figure 5 shows in a schematic way the final single strand DNA library obtained according to a method of generating a massively parallel sequencing library disclosed herein applied to a fragment of DRS-WGA following the steps shown in figure 4; moreover, figure 5 illustrates the final sequenced ssDNA library and Custom sequencing primers designed; starting from few hundred tumor cells digitally sorted from FFPE with DEPArray system (Bolognesi et al.) it is generated a DRS-WGA library;
Figure 6 shows the sequencing results of a Low-pass Whole Genome Sequencing performed starting from few hundred tumor cells digitally sorted from FFPE with DEPArray system on a DNA library prepared according to the disclosed method and sequenced by PGM platform;

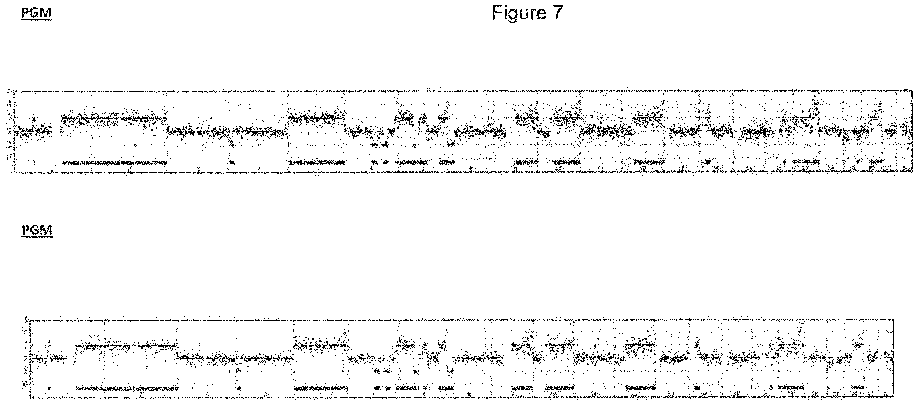

Figure 7 shows the sequencing results of Low-pass Whole Genome Sequencing performed by PGM protocol on DNA libraries prepared on two different tumor cells;

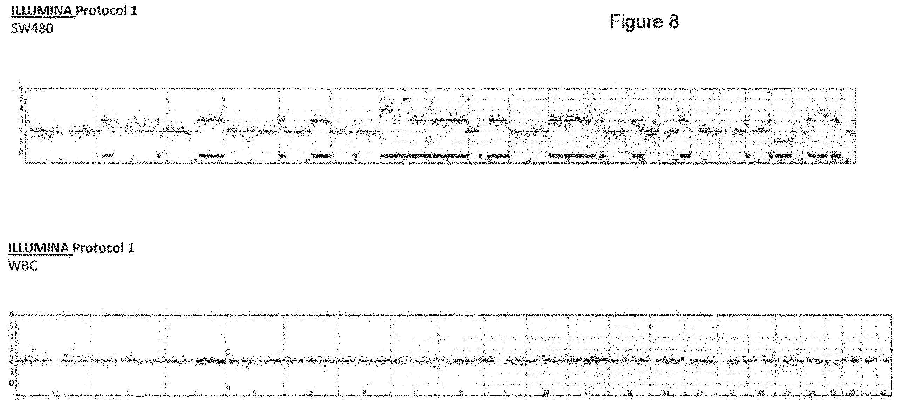

Figure 8 shows the sequencing results of a Low-pass Whole Genome Sequencing performed by a ILLUMINA protocol 1 on DNA libraries prepared according to the disclosed method and compares the results obtained from a normal WBC cell and an abnormal (tumoral) cell; and

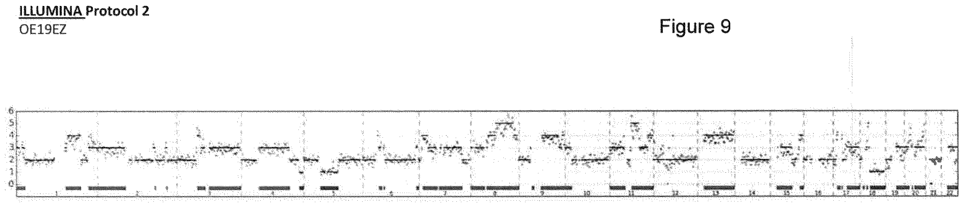

Figure 9 shows the sequencing results of a Low-pass Whole Genome Sequencing performed by a ILLUMINA protocol 2 according to one aspect of the invention on DNA libraries prepared according to the disclosed method.

### Detailed Description

### Definitions

Unless defined otherwise, all technical and scientific terms used herein have the same meaning as commonly understood by one of ordinary skill in the art to which this invention pertains. Unless mentioned otherwise, the techniques described herein for use with the invention are standard methodologies well known to persons of ordinary skill in the art.

By the term "Digestion site (DS)" or "Restriction Site (RS)" it is intended the sequence of nucleotides (typically 4-8 base pairs (bp) in length) along a DNA molecule recognized by the restriction enzyme as to where it cuts along the polynucleotide chain.

By the term "Cleavage site" it is intended the site in a polynucleotide chain as to where the restriction enzyme cleaves nucleotides by hydrolyzing the phosphodiester bond between them.

By the term "Amplicon" it is intended a region of DNA produced by a PCR amplification.

By the term "DRS-WGA Amplicon" or -in short- "WGA amplicon" it is intended a DNA fragment amplified during DRS-WGA, comprising a DNA sequence between two RS flanked by the ligated Adaptors.

By the term "Original DNA" it is intended the genomic DNA (gDNA) prior to amplification with the DRS-WGA.

By the term "Adaptor" or "WGA Adaptor" or "WGA PCR Primer" or "WGA library universal sequence adaptor" it is intended the additional oligonucleotide ligated to each fragment generated by the action of the restriction enzyme, in case of DRS-WGA, or the known polynucleotide sequence present at 5' section of each molecule of the WGA DNA library as a result of extension and PCR process, in case of MALBAC.

By the term "Copy Number Alteration (CNA)" it is intended a somatic change in copy-numbers of a genomic region, defined in general with respect to the same individual genome.

By the term "Copy Number Variation (CNV) " it is intended a germline variant in copy-numbers of a genomic region, defined in general with respect to a reference genome. Throughout the description CNA and CNV may be used interchangeably, as most of the reasoning can be applied to both situations. It should be intended that each of those terms refers to both situations, unless the contrary is specified.

By the term "Massive-parallel next generation sequencing (NGS)" it is intended a method of sequencing DNA comprising the creation of a library of DNA molecules spatially and/or time separated, clonally sequenced (with or without prior clonal amplification). Examples include Illumina platform (Illumina Inc), Ion Torrent platform (ThermoFisher Scientific Inc), Pacific Biosciences platform, MinION (Oxford Nanopore Technologies Ltd).

By the term "Target sequence" it is intended a region of interest on the original DNA.

By the term "Primary WGA DNA library (pWGAlib)" it is indented a DNA library obtained from a WGA reaction.

By the term "Multiple Annealing and Looping Based Amplification Cycles (MALBAC)" it is intended a quasilinear whole genome amplification method (Zong et al., Genome-wide detection of single-nucleotide and copy-number variations of a single human cell, Science. 2012 Dec 21;338(6114):1622-6. doi: 10.1126/science.1229164.). MALBAC primers have a 8 nucleotides 3' random sequence, to hybridize to the template, and a 27 nucleotides 5' common sequence (GTG AGT GAT GGT TGA GGT AGT GTG GAG). After first extension, semiamplicons are used as templates for another extension yielding a full amplicon which has complementary 5' and 3' ends. Following few cycles of quasi-linear amplification, full amplicon can be exponentially amplified with subsequent PCR cycles.

By the term "DNA library Purification" it is intended a process whereby the DNA library material is separated from unwanted reaction components such as enzymes, dNTPs, salts and/or other molecules which are not part of the desired DNA library. Example of DNA library purification processes are purification with magnetic bead-based technology such as Agencourt AMPure XP or solid-phase reversible immobilization (SPRI)-beads from Beckman Coulter or with spin column purification such as Amicon spin-columns from Merck Millipore.

By the term "DNA library Size selection" it is intended a process whereby the base-pair distribution of different fragments composing the DNA library is altered. In general, a portion of DNA library included in a certain range is substantially retained whereas DNA library components outside of that range are substantially discarded. Examples of DNA library Size selection processes are excision of electrophoretic gels (e.g. ThermoFisher Scientific E-gel), or double purification with magnetic beads-based purification system (e.g. Beckman Coulter SPRI-beads).

By the term "DNA library Selection" it is intended a process whereby either DNA library Purification or DNA library Size selection or both are carried out.

By the term "NGS Re-amplification" it is intended a PCR reaction where all or a substantial portion of the primary WGA DNA library is further amplified. The term NGS may be omitted for simplicity throughout the text, and reference will be made simply to "re-amplification".

By the term "Sequencing adaptor (SA)" it is intended one or more molecules which are instrumental for sequencing the DNA insert, each molecule may comprise none, one or more of the following: a polynucleotide sequence, a functional group. In particular, it is intended a polynucleotide sequence which is required to be present in a massively parallel sequencing library in order for the sequencer to generate correctly an output sequence, but which does not carry information, (as non-limiting examples: a polynucleotide sequence to hybridize a ssDNA to a flow-cell, in case of Illumina sequencing, or to an ion-sphere, in case of Ion Torrent sequencing, or a polynucleotide sequence required to initiate a sequencing-by-synthesis reaction).

By the term "Sequencing barcode" it is intended a polynucleotide sequence which, when sequenced within one sequencer read, allows that read to be assigned to a specific sample associated with that barcode.

By the term "functional for a selected sequencing platform" it is intended a polynucleotide sequence which has to be employed by the sequencing platform during the sequencing process (e.g. a barcode or a sequencing adaptor).

By the term "Low-pass whole genome sequencing" it is intended a whole genome sequencing at a mean sequencing depth lower than 1.

By the term "Mean sequencing depth" it is intended here, on a per-sample basis, the total of number of bases sequenced, mapped to the reference genome divided by the total reference genome size. The total number of bases sequenced and mapped can be approximated to the number of mapped reads times the average read length.

By the term "double-stranded DNA (dsDNA) " it is intended, according to base pairing rules (A with T, and C with G), two separate polynucleotide complementary strands hydrogen bonded by binding the nitrogenous bases of the two. Single-stranded DNA (ssDNA) : The two strands of DNA can form two single-stranded DNA molecules, i.e. a DNA molecule composed of two ssDNA molecule coupled with Watson-Crick base pairing.

By the term "single-stranded DNA (ssDNA) " it is intended a polynucleotide strand e.g. derived from a double-stranded DNA or which can pairs with a complementary single-stranded DNA, i.e. a polynucleotide DNA molecule consisting of only a single strand contrary to the typical two strands of nucleotides in helical form.

By "equalizing" it is intended the act of adjusting the concentration of one or more samples to make them equal.

By "normalizing" it is intended the act of adjusting the concentration of one or more samples to make them correspond to a desired proportion between them (equalizing being the special case where the proportion is 1) . In the description, for the sake of simplicity, the terms normalizing and equalizing will be used indifferently as they are obviously conceptually identical.

By "paramagnetic beads" it is intended streptavidin conjugated magnetic beads (e.g. Dynabeads^{®} MyOne^{™} Streptavidin C1, ThermoFisher Scientific). The expression "designed conditions" when referring to incubation of the paramagnetic beads refers to the conditions required for the activation step, which consists in washing the streptavidin conjugated magnetic beads two times with the following buffer: 10 mM Tris-HCl (pH 7.5), 1 mM EDTA, 2 M NaCl.

### Workflows

The following table summarizes some possible workflows:

**TABLE 1**

| Step | wf1 | wf2 | wf3 | wf4 | wf5 | wf6 | wf7 |
|---|---|---|---|---|---|---|---|
| Purify/Size Select | O | O | O | O | O | O | X |
| NGS Re-Amp | SA | BC+SA | BC+SA | SA | BC+SA | BC | BC+SA |
| Purify | | | X | | | | X |
| Quantitate | | | X | | | | X |
| Pool | | X | X | | | | X |
| Size Select | X | X | X | | | | |
| Purify | O | O | O | O | O | O | |
| Sequence | X | X | X | X | X | X | X |

| | | | | | | | |
|---|---|---|---|---|---|---|---|
| **Legenda:** O=optional step, SA=introduction of Sequencing Adaptor(s), BC=introduction of BarCodes, X=needed step, wf=workflow | | | | | | | |

### Process input material

All the present description refers to a primary WGA DNA library. The same workflows may apply to primary WGA DNA library which were further subjected to additional processes, such as for example, dsDNA synthesis, or library re-amplification with standard WGA primers (e.g. as possible with *Ampli1^{™}* ReAmp/ds kit, Menarini Silicon Biosystems spa, Italy). For the sake of simplicity we refer here only to primary WGA DNA libraries, without having regard of those additional processes. It should be intended that all those kind of input samples may be used as suitable sample input, also for what reported in the claim.

### Initial purification

When non-negligible amounts of primary WGA primers are present in the primary WGA output product, it may be of advantage to have an initial DNA library Selection including a DNA library Purification. In fact, since the primers include, at the 3' end, a sequence corresponding to the common sequence found in primary WGA primers, the presence of non-negligible amounts of residual primary WGA primers may compete with the re-amplification primers used to obtain the massively parallel sequencing library, decreasing the yield of DNA-library molecules having -as desired- the reamplification primer(s) (or their reverse complementary) on both ends.

### Quantitation for equalization of number of reads across samples

When the variations in amount of re-amplified DNA library are relatively large among samples to be pooled and sequenced together, it may be of advantage to quantitate the amount of DNA library from each sample, in order to aliquot those libraries and equalize the number of reads sequenced for each sample.

### Mismatch between sequencer read length and WGA size peak can result in imprecise equalization

Several massively parallel sequencers (including Ion Torrent and Illumina platforms) employ sequencing of DNA fragments having a size distribution peak comprised between 50 and 800 bp, such as for example those having a distribution peaking at 150bp, 200bp, 400bp, 650 bp according to the different chemistries used. As pWGAlib size distribution can have a peak of larger fragments, such as about 1 kbp, and much smaller amounts of DNA at 150 bp, 200 bp, 400 bp, the quantitation of re-amplified DNA library amounts in the desired range may be imprecise if carried out on the bulk re-amplified DNA library without prior sizeselection of the desired fragments range. As a result, the DNA quantitation in bulk and equalization of various samples in the pool may result in relatively large variations of the actual number of reads per sample, as the number of fragments within the sequencer size-range varies stochastically due to the imprecision in the distribution of DNA fragments in the library (thus, even perfectly equalized total amounts of DNA library result in significant variations of number of sequenced fragments).

### Increase amount of DNA library within sequencer read-length to improve equalization [by size selection prior to re-amplification]

When the primary WGA product size distribution should be altered to increase the proportion of amount of DNA library within the sequencer read length range with respect to total DNA library, it may be of advantage to have an initial DNA library selection comprising a DNA library Size selection.

### [by preferential re-amplification]

Alternatively, or in addition to, it may be of advantage to carry out the re-amplification reaction under conditions favoring the preferential amplification of DNA library fragments in the desired range.

### Preferential re-amplification by polymerase choice or extension cycle shortening

Reaction conditions favoring shorter fragments may comprise re-amplification PCR reaction with a polymerase preferentially amplifying shorter fragments, or initial PCR cycles whereby a shorter extension phase prevents long fragments to be replicated to their full length, generating incomplete library fragments. Incomplete library fragments lack the 3' end portion reverse-complementary to the reamplification primer(s) 3' section and thus exclude the fragment from further replication steps with said reamplification primer(s), interrupting the exponential amplification of the incomplete fragment, consenting the generation of only a linear (with cycles) number of incomplete amplification fragments originated by the longer primary WGA DNA library fragments.

### Example of workflows according to TABLE 1

Wf1) may be applied to LPWGS of a WGA library on IonTorrent PGM (e.g. on a 314 chip, processing a single sample which does not require sample barcodes). The reamplification with two primers allows the introduction of the two sequencing adaptors, without barcodes.

Wf2) may be applied to LPWGS of multiple primary WGA samples on Ion Torrent PGM or Illumina MiSeq, when the original input samples for the primary WGA derive from homogenous types of unamplified material, e.g. single-cells, which underwent through the same treatment (e.g. fresh or fixed), non-apoptotic. Thus no quantitation is necessary as the primary WGA yield is roughly the same across all. Barcoded, sequencer-adapted libraries are pooled, then size selected to isolate fragments with the appropriate size within sequencer read length, purified and sequenced. If size selection is carried out by gel, a subsequent purification is carried out. If size selection is carried out for example with double-sided SPRI-bead purification, the resulting output is already purified and no further purification steps are necessary.

Wf3) may be applied to LPWGS of multiple primary WGA samples on Ion Torrent PGM or Illumina MiSeq where the original input samples for the primary WGA derive from non-homogenous types of unamplified material. E.g. part single-cells, part cell pools, which underwent through different treatments (e.g. some fresh some fixed), with different original DNA quality (some non-apoptotic, some apoptotic, with heterogeneous genome integrity indexes -see Polzer et al. EMBO Mol Med. 2014 Oct 30;6(11) :1371-86). Thus, quantitation is necessary as the primary WGA yield may differ significantly across samples. With respect to Wf2, a quantitation is carried out. Prior to quantitation it is of advantage to purify in order to make the quantitation step more reliable as, e.g. residual primers and dNTPs or primer dimers are removed and do not affect the quantitation.

Barcoded, sequencer-adapted libraries are pooled, then size selected to isolate fragments with the appropriate size within sequencer read length, purified and sequenced. If size selection is carried out by gel, a subsequent purification is carried out. If size selection is carried out for example with double-sided SPRI-bead purification, the resulting output is already purified and no further purification steps are necessary.

Wf4) may be applied to the preparation of a massively parallel sequencing library for Oxford Nanopore sequencing. Since the Nanopore can accommodate longer read-lengths, size selection may be unnecessary, and sequencing can be carried out on substantially all fragment lengths in the library.

Wf5) may be applied to the preparation of multiple massively parallel sequencing libraries for Oxford Nanopore sequencing. With respect to wf4, the re-amplification primers further include a sample barcode for multiplexing more samples in the same run. Since the Nanopore can accommodate longer read-lengths, size selection may be unnecessary.

Wf6) may be applied to the preparation of multiple massively parallel sequencing libraries for an Oxford Nanopore sequencer not requiring the addition of special-purpose adaptors. With respect to wf5, the reamplification primers do not include a sequencing adaptor but only a sample barcode for multiplexing more samples in the same run. Since the Nanopore can accommodate longer read-lengths, size selection may be unnecessary.

Wf7) may be applied to the preparation of multiple massively parallel libraries for sequencing of DRS-WGA DNA libraries obtained from non-homogenous samples following heterogeneous treatments and having different DNA quality on a shorter read-length system, such as IonProton using sequencing 200bp chemistry. Since the amount of primary WGA DNA library around 200bp is very small compared to the total DNA in the primary WGA DNA library, it may be of advantage to carry out a size selection eliminating all or substantially all pWGAlib fragments outside of the sequencing read-length, enriching for pWGAlib fragments around 200bp.

Re-amplification is then carried out with re-amplification primers including Barcode and sequencing adaptors compatible with IonProton system. Re-amplification product is thus purified and quantitated for each sample, and different aliquots of different samples are pooled together so as to equalize the number of reads for each sample barcode, and then sequenced to carry out LPWGS.

For those with ordinary skill in the art it is apparent that different combinations of the steps included in the workflows as mentioned above are possible without departing from the scope of the invention, which hinges in the re-amplification of the primary WGA DNA library with special primers as disclosed herein.

### Massively parallel sequencing library preparation from a WGA product

In the present disclosure, a method is provided comprising the steps of
a. providing a primary WGA DNA library (pWGAlib) including fragments comprising a known 5' WGA sequence section (5SS), a middle WGA sequence section (MSS), and a known 3' WGA sequence section (3SS) reverse complementary to the known 5' WGA sequence section, the known 5' WGA sequence section (5SS) comprising a WGA library universal sequence adaptor, and the middle WGA sequence section (MSS) comprising at least an insert section (IS) corresponding to a DNA sequence of the original unamplified DNA prior to WGA, the middle WGA sequence optionally comprising, in addition, a flanking 5' intermediate section (F5) and/or a flanking 3' intermediate section (F3);
b. re-amplifying the primary WGA DNA library using at least one first primer (1PR) and at least one second primer (2PR); wherein
   the at least one first primer (1PR) comprises a first primer 5' section (1PR5S) and a first primer 3' section (1PR3S), the first primer 5' section (1PR5S) comprising at least one first sequencing adaptor (1PR5SA) and at least one first sequencing barcode (1PR5BC) in 3' position of the at least one first sequencing adaptor (1PR5SA) and in 5' position of the first primer 3' section (1PR3S), and the first primer 3' section (1PR3S) hybridizing to either the known 5' sequence section (5SS) or the known 3' sequence section (3SS);
   the at least one second primer (2PR) comprises a second primer 5' section (2PR5S) and a second primer 3' section (2PR3S), the second primer 5' section (2PR5S) comprising at least one second sequencing adaptor (2PR5SA) different from the at least one first sequencing adaptor (1PR5SA), and the second primer 3' section (2PR3S) hybridizing to either the known 5' sequence section (5SS) or the known 3' sequence section (3SS).

The known 5' sequence section (5SS) preferably consists of a WGA library universal sequence adaptor. As an example, DRS-WGA (such as Menarini Silicon Biosystems *Ampli*1*^{™}* WGA kit) as well as MALBAC (Yikon Genomics), produce pWGAlib with known 3' sequence section reverse complementary of said known 5' sequence section as requested for the input of the method disclosed herein.

The WGA library universal sequence adaptor is therefore preferably a DRS-WGA library universal sequence adaptor (e.g. SEQ ID NO: 282) or a MALBAC library universal sequence adaptor (e.g. SEQ ID NO: 283), more preferably a DRS-WGA library universal sequence adaptor.

Preferably, the second primer (2PR) further comprises at least one second sequencing barcode (2PR5BC), in 3' position of the at least one second sequencing adaptor (2PR5SA) and in 5' position of said second primer 3' section (2PR3S).

Owing to the presence of the sequencing barcodes, a method for low-pass whole genome sequencing is carried out according to one embodiment of the invention, comprising the steps of:
c. providing a plurality of barcoded, massively-parallel sequencing libraries and pooling samples obtained using different sequencing barcodes (BC);
d. sequencing the pooled library.

The step of pooling samples using different sequencing barcodes (BC) further comprises the steps of:
e. quantitating the DNA in each of said barcoded, massively-parallel sequencing libraries;
f. normalizing the amount of barcoded, massively-parallel sequencing libraries.

The step of pooling samples using different sequencing barcodes (BC) further comprises the step of selecting DNA fragments comprised within at least one selected range of base pairs. Such selected range of base pairs is centered on different values in view of the downstream selection of the sequencing platform. E.g. for the Illumina sequencing platform, the range of base pairs is centered on 650 bp and preferably on 400 bp. For other sequencing platforms, e.g. Ion Torrent, the range of base pairs is centered on 400 bp and preferably on 200 bp and more preferably on 150 bp or on 100 bp or on 50 bp.

According to one further embodiment of the invention the method for low-pass whole genome sequencing as referred to above further comprises the step of selecting DNA fragments comprising both the first sequencing adaptor and the second sequencing adaptors.

Preferably, the step of selecting DNA fragments comprising said first sequencing adaptor and said second sequencing adaptors is carried out by contacting the massively parallel sequencing library to at least one solid phase consisting in/comprising e.g. functionalized paramagnetic beads. In one embodiment of the method of the invention, the paramagnetic beads are functionalized with a streptavidin coating.

In one method for low-pass whole genome sequencing according to the invention one of the at least one first primer (1PR) and the at least one second primer (2PR) are biotinylated at the 5' end, and selected fragments are obtained eluting from the beads non-biotinylated ssDNA fragments.

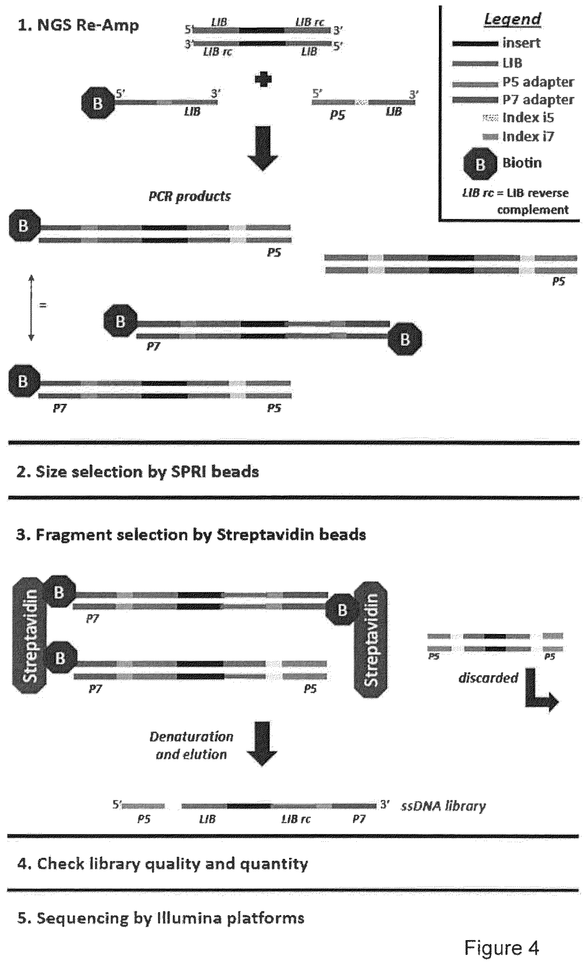

As can be seen from Figure 4, in the above case the reamplified WGA dsDNA library comprises: 1) non-biotinylated dsDNA fragments, dsDNA fragments biotinylated on one strand and dsDNA fragments biotinylated on both strands. The method of the invention comprises the further steps of:
g. incubating the re-amplified WGA dsDNA library with the functionalized paramagnetic beads under designed conditions which cause covalent binding between biotin and streptavidin allocated in the functionalized paramagnetic beads;
h. washing out unbound non biotinylated dsDNA fragments;
i. eluting from the functionalized paramagnetic beads the retained ssDNA fragments.

There is also disclosed a massively parallel sequencing library preparation kit comprising at least:
- one first primer (1PR) comprising a first primer 5' section (1PR5S) and a first primer 3' section (1PR3S),
   the first primer 5' section (1PR5S) comprising at least one first sequencing adaptor (1PR5SA) and at least one first sequencing barcode (1PR5BC) in 3' position of the at least one first sequencing adaptor (1PR5SA) and in 5' position of the first primer 3' section (1PR3S), and the first primer 3' section (1PR3S) hybridizing to either a known 5' sequence section (5SS) comprising a WGA library universal sequence adaptor or a known 3' sequence section (3SS) reverse complementary to the known 5' sequence section of fragments of a primary WGA DNA library (pWGAlib), the fragments further comprising a middle sequence section (MSS) 3' of the known 5' sequence section (5SS) and 5' of the known 3' sequence section (3SS);
- one second primer (2PR) comprising a second primer 5' section (2PR5S) and a second 3' section (2PR3S), the second primer 5' section (2PR5S) comprising at least one second sequencing adaptor (2PR5SA) different from the at least one first sequencing adaptor (1PR5SA), the second 3' section hybridizing to either the known 5' sequence section (5SS) or the known 3' sequence section (3SS) of the fragments

In particular, the massively parallel sequencing library preparation kit comprises:
a) the primer SEQ ID NO:97 (Table 2) and one or more primers selected from the group consisting of SEQ ID NO:1 to SEQ ID NO: 96 (Table 2);
   or
b) the primer of SEQ ID NO:194 (Table 2) and one or more primers selected from the group consisting of SEQ ID NO:98 to SEQ ID NO:193 (Table 2);
   or
c) at least one primer selected from the group consisting of SEQ ID NO:195 to SEQ ID NO:202 (Table 4), and at least one primer selected from the group consisting of SEQ ID NO:203 to SEQ ID NO:214 (Table 4);
   or
d) at least one primer selected from the group consisting of SEQ ID NO:215 to SEQ ID NO:222 (Table 6), and at least one primer selected from the group consisting of SEQ ID NO:223 to SEQ ID NO:234 (Table 6);
   or
e) at least one primer selected from the group consisting of SEQ ID NO:235 to SEQ ID NO:242 (Table 7), and at least one primer selected from the group consisting of SEQ ID NO:243 to SEQ ID NO:254 (Table 7);
   or
f) at least one primer selected from the group consisting of SEQ ID NO:259 to SEQ ID NO:266 (Table 8), and at least one primer selected from the group consisting of SEQ ID NO:267 to SEQ ID NO:278 (Table 8).

The massively parallel sequencing library preparation kit may comprise:
- at least one primer selected from the group consisting of SEQ ID NO:235 to SEQ ID NO:242 (Table 7), and at least one primer selected from the group consisting of SEQ ID NO:243 to SEQ ID NO:254 (Table 7); a custom sequencing primer of SEQ ID NO:255; and a primer of SEQ ID NO:256 or SEQ ID NO:258; or
- at least one primer selected from the group consisting of SEQ ID NO:259 to SEQ ID NO:266 (Table 8), at least one primer selected from the group consisting of SEQ ID NO:267 to SEQ ID NO:278 (Table 8); and primers of SEQ ID NO:279 and SEQ ID NO:280;
designed to carry out an optimum single read sequencing process.

The above kit may further comprise a primer selected from SEQ ID NO:257 (Table 7) and SEQ ID NO:281 (Table 8) designed to carry out an optimum Paired-End sequencing process in a selected sequencing platform.

Preferably, the massively parallel sequencing library preparation kit further comprises a thermostable DNA polymerase.

The present invention finally relates also to a method for genome-wide copy number profiling, comprising the steps of
a. sequencing a DNA library developed using the sequencing library preparation kit as described above,
b. analysing the sequencing read density across different regions of the genome,
c. determining a copy-number value for the regions of the genome by comparing the number of reads in that region with respect to the number of reads expected in the same for a reference genome.

### Low-Pass Whole Genome Sequencing from single CTCs

CNA profiling by LPWGS is more tolerant to lower genome-integrity index, where aCGH may fail to give results clean enough. In fact, aCGH probes are designed for fixed positions in the genome. If those positions stochastically fail to amplify due to cross linking of DNA, the corresponding probe will not generate the appropriate amount of signal following hybridization, resulting in a noisy pixel in the signal ratio between test DNA and reference DNA.

On the contrary, using LPWGS, fragments are based only on size selection. If certain fragments stochastically do not amplify due to e.g. crosslinking of DNA or breaks induced by apoptosis, there may still be additional fragments of the same size amenable to amplification in nearby genomic regions falling into the same low-pass bin. Accordingly the signal-to-noise is more resilient to genome-integrity index of the library, as e.g. clearly shown in figures from 6 to 9.

### Massively-parallel sequencing library preparation from DRS-WGA

Size selection, implies a subsampling of the genome within regions comprised of DRS-WGA fragments of substantially the same length (net of adaptors insertion) as the sequencing library base-pair size.

Nevertheless it has been surprisingly found that these subsampling does not impact the quality of the copy-number profile, even when using standard algorithms for copy-number variant calling.

Advantageously the DRS-WGA is selected (as Ampi1^{™} WGA kit), having a TTAA deterministic restriction site. In this way, shorter fragments are denser in low GC content regions of the genome, and the fragment density correlates negatively with higher GC content.

### Low-Pass Whole Genome Sequencing from minute amounts of digitally sorted FFPE cells

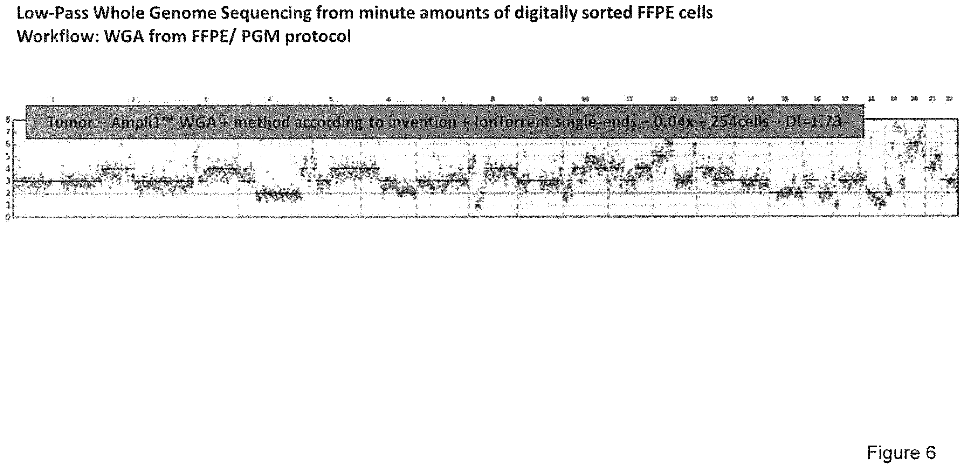

Starting from few hundred tumor cells digitally sorted from FFPE with DEPArray system (Bolognesi et al.) we generated a DRS-WGA library. The library was used to generate a massively parallel sequencing library for Ion/PGM according to the invention, as shown in Figure 6. The massively parallel library was sequenced at <0,05 mean depth.

### Example 1:

### Protocol for LPWGS on Ion Torrent PGM following DRS-WGA

### 1) Deterministic-Restriction Site Whole genome amplification (DRS-WGA)

Single cell DNA was amplified using the *Ampli1^{™}* WGA Kit (Silicon Biosystems) according to the manufacturer's instructions.

The *Ampli1*^{™}WGA Kit is designed to provide whole genome amplification from DNA obtained from one single cell. Following cell lysis, DNA is digested with a restriction enzyme, preferably MseI, and a universal adaptor sequence are ligated to DNA fragments. Amplification is mediated by a single specific PCR primer for all generated fragments, with a range size of 200-1,000 bp in length, which are distributed across the genome.

### 2) Re-amplification of the WGA products

Five µL of WGA-amplified DNA are diluted by addition of 5 µL of Nuclease-Free Water and purified using Agencourt AMPure XP system (Beckman Coulter) in order to remove unbound oligonucleotides and excess nucleotides, salts and enzymes.

The beads-based DNA purification was performed according to the following protocol: 18 µL of beads (1.8X sample volume) were added to each sample. Beads and reaction products were mixed by briefly vortexing and then spun-down to collect the droplets. Mixed reactions were then incubated off-magnet for 15 min at RT, after which they were then transferred to a DynaMag-96 Side magnet (Life Technologies) and left to stand for 5 min. Supernatant were discarded and beads washed with 150 µL of freshly made 80% EtOH. After a second round of EtOH washing, beads were allowed to dry on the magnet for 5-10 min. Dried beads were then resuspended off-magnet in 15 µL of LowTE buffer and incubated for 10 min, followed by 5 min incubation on-magnet. Twelve microliters of the eluate were transferred to another tube and subsequently quantified by dsDNA HS Assay on the Qubit^{®} 2.0 Fluorometer in order to prepare aliquots of 10 µL containing 25 ng of WGA-purified DNA.

Barcoded re-amplification was performed in a volume of 50 µl using *Ampli1^{™}* PCR Kit (Menarini Silicon Biosystems). Each PCR reaction was composed as follows: 5 µl *Ampli1^{™}* PCR Reaction Buffer (10X), 1µL of 25 µM of one primer of SEQ ID NO:1 to SEQ ID NO:96
[1] **(5'-CCATCTCATCCCTGCGTGTCTCCGACTCAG[BC-]AGTGGGATTCCTGCTGTCAGT-3')**
   where [BC] = Barcode sequence, 1µL of 25 µM of the SEQ ID NO:97 primer
**[2] (5'-CCTCTCTATGGGCAGTCGGTGATAGTGGGATTCCTGCTGTCAGT-3')**
   1.75 µl *Ampli1^{™}* PCR dNTPs (10 mM), 1.25 µl BSA, 0.5 *Ampli1^{™}* PCR Taq Polymerase, 37.5 µl of *Ampli1^{™}* Water and 25 ng of the WGA-purified DNA.

Applied Biosystems^{®} 2720 Thermal Cycler was set as follows: 95°C for 4 min, 1 cycle of 95°C for 30 sec, 60°C for 30 sec, 72°C for 2 min, 10 cycles of 95°C for 30 sec, 60°C for 30 sec, 72°C for 2 min (extended by 20 sec/cycle) and final extension at 72° C for 7 min.

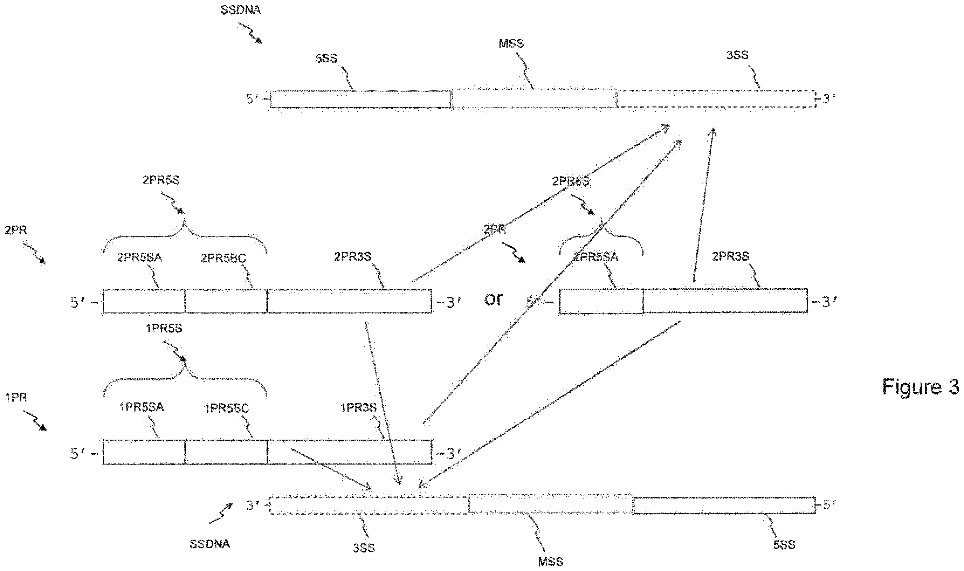

Figure 3 shows schematically the re-amplification process. Barcoded re-amplified WGA products were purified with 1.8× (90 µl) AMPure XP beads and eluted in 35 µl of Low TE buffer according to the steps described above.

### 3) Size selection

Barcoded re-amplified WGA products, correspondent to a fragment library with provided Ion Torrent adapters, were qualified by Agilent DNA 7500 Kit on the 2100 Bioanalyzer^{®} (Agilent) and quantified using Qubit^{®} dsDNA HS Assay Kit in order to obtain a final pool.

The equimolar pool was created by combining the same amount of individual 7 libraries with different A-LIB-BC-X adapter, producing the final pool with the concentration of 34 ng/µL in a final volume of 42 µL. The concentration of the pool was confirmed by the Qubit^{®} method.

E-Gel^{®} SizeSelect^{™} system in combination with Size Select 2% precast agarose gel (Invitrogen) has been used for the size selection of fragments of interest, according to the manufacturer's instructions.

Twenty µL of the final pool were loaded on two lanes of an E-gel and using a size standard (50bp DNA Ladder, Invitrogen), a section range between 300 to 400 bp has been selected from the gel.

Following size selection, the clean up was performed with 1.8x (90 µl) AMPure XP beads. Final library was eluted in 30 µl of Low TE buffer according to the steps described above and evaluated using a 2100 Bioanalyzer High Sensitivity Chip (Agilent Technologies).

### 4) Ion Torrent PGM sequencing

Template preparation was performed according to the Ion PGM^{™} Hi-Q OT2 kit-400bp user guide.

Briefly, Library fragments were clonally amplified onto Ion Sphere Particles (ISPs) through emulsion PCR and then enriched for template-positive ISPs. PGM emulsion PCR reactions were performed with the Ion PGM^{™} Hi-Q OT2 kit (Life Technologies) and emulsions and amplifications were generated utilizing the Ion OneTouch Instrument (Life Technologies). Following recovery, enrichment was performed by selectively binding the ISPs containing amplified library fragments to streptavidin coated magnetic beads, removing empty ISPs through washing steps, and denaturing the library strands to allow collection of the template-positive ISPs.

The described enrichment steps were accomplished using the Life Technologies ES System (Life Technologies).

Ion 318v2^{™} Chip was loaded following "Simplified Ion PGM^{™} Chip loading with the Ion PGM^{™} weighted chip bucket" protocol instructions (MAN0007517).

All samples were processed by Ion Personal Genome Machine (PGM) (Life Technologies) using the Ion PGM^{™} Hi-Q^{™} Sequencing Kit (Life Technologies) and setting the 520 flow run format.

Finally, the sequenced fragments were assigned to specific samples based on their unique barcode.

**TABLE 2**

| NGS re-amplification primers for Ion Torrent platform (PGM/Proton) | | |
|---|---|---|
| a) SEQ ID NO list_first_primer_[PGM/DRS-WGA] | | |
| **SEQ ID NO** | **Primer name** | **Primer sequence** |
| SEQ ID NO:1 | A -BC-LIB_1 | 5'-CCATCTCATCCCTGCGTGTCTCCGACTCAGCTAAGGTAACAGTGGGATTCCTGCTGTCAGT-3' |
| SEQ ID NO:2 | A -BC-LIB_2 | 5'-CCATCTCATCCCTGCGTGTCTCCGACTCAGTAAGGAGAACAGTGGGATTCCTGCTGTCAGT-3' |
| SEQ ID NO:3 | A -BC-LIB_3 | 5'-CCATCTCATCCCTGCGTGTCTCCGACTCAGAAGAGGATTCAGTGGGATTCCTGCTGTCAGT-3' |
| SEQ ID NO:4 | A -BC-LIB_4 | 5'-CCATCTCATCCCTGCGTGTCTCCGACTCAGTACCAAGATCAGTGGGATTCCTGCTGTCAGT-3' |
| SEQ ID NO:5 | A -BC-LIB_{_}5 | 5'-CCATCTCATCCCTGCGTGTCTCCGACTCAGCAGAAGGAACAGTGGGATTCCTGCTGTCAGT-3' |
| SEQ ID NO:6 | A -BC-LIB_6 | 5'-CCATCTCATCCCTGCGTGTCTCCGACTCAGCTGCAAGTTCAGTGGGATTCCTGCTGTCAGT-3' |
| SEQ ID NO:7 | A -BC-LIB_7 | 5'-CCATCTCATCCCTGCGTGTCTCCGACTCAGTTCGTGATTCAGTGGGATTCCTGCTGTCAGT-3' |
| SEQ ID NO:8 | A -BC-LIB_8 | 5'-CCATCTCATCCCTGCGTGTCTCCGACTCAGTTCCGATAACAGTGGGATTCCTGCTGTCAGT-3' |
| SEQ ID NO:9 | A -BC-LIB_9 | 5'-CCATCTCATCCCTGCGTGTCTCCGACTCAGTGAGCGGAACAGTGGGATTCCTGCTGTCAGT-3' |
| SEQ ID NO:10 | A -BC-LIB_10 | 5'-CCATCTCATCCCTGCGTGTCTCCGACTCAGCTGACCGAACAGTGGGATTCCTGCTGTCAGT-3' |
| SEQ ID NO:11 | A -BC-LIB_11 | 5'-CCATCTCATCCCTGCGTGTCTCCGACTCAGTCCTCGAATCAGTGGGATTCCTGCTGTCAGT-3' |
| SEQ ID NO:12 | A -BC-LIB_12 | 5'-CCATCTCATCCCTGCGTGTCTCCGACTCAGTAGGTGGTTCAGTGGGATTCCTGCTGTCAGT-3' |
| SEQ ID NO:13 | A -BC-LIB_13 | 5'-CCATCTCATCCCTGCGTGTCTCCGACTCAGTCTAACGGACAGTGGGATTCCTGCTGTCAGT-3' |
| SEQ ID NO:14 | A -BC-LIB_14 | 5'-CCATCTCATCCCTGCGTGTCTCCGACTCAGTTGGAGTGTCAGTGGGATTCCTGCTGTCAGT-3' |
| SEQ ID NO:15 | A -BC-LIB_15 | 5'-CCATCTCATCCCTGCGTGTCTCCGACTCAGTCTAGAGGTCAGTGGGATTCCTGCTGTCAGT-3' |
| SEQ ID NO:16 | A -BC-LIB_16 | 5'-CCATCTCATCCCTGCGTGTCTCCGACTCAGTCTGGATGACAGTGGGATTCCTGCTGTCAGT-3' |
| SEQ ID NO:17 | A -BC-LIB_17 | 5'-CCATCTCATCCCTGCGTGTCTCCGACTCAGTCTATTCGTCAGTGGGATTCCTGCTGTCAGT-3' |
| SEQ ID NO:18 | A -BC-LIB_18 | 5'-CCATCTCATCCCTGCGTGTCTCCGACTCAGAGGCAATTGCAGTGGGATTCCTGCTGTCAGT-3' |
| SEQ ID NO:19 | A -BC-LIB_19 | 5'-CCATCTCATCCCTGCGTGTCTCCGACTCAGTTAGTCGGACAGTGGGATTCCTGCTGTCAGT-3' |
| SEQ ID NO:20 | A -BC-LIB_20 | 5'-CCATCTCATCCCTGCGTGTCTCCGACTCAGCAGATCCATCAGTGGGATTCCTGCTGTCAGT-3' |
| SEQ ID NO:21 | A -BC-LIB_21 | 5'-CCATCTCATCCCTGCGTGTCTCCGACTCAGTCGCAATTACAGTGGGATTCCTGCTGTCAGT-3' |
| SEQ ID NO:22 | A -BC-LIB_22 | 5'-CCATCTCATCCCTGCGTGTCTCCGACTCAGTTCGAGACGCAGTGGGATTCCTGCTGTCAGT-3' |
| SEQ ID NO:23 | A -BC-LIB_23 | 5'-CCATCTCATCCCTGCGTGTCTCCGACTCAGTGCCACGAACAGTGGGATTCCTGCTGTCAGT-3' |
| SEQ ID NO:24 | A -BC-LIB_24 | 5'-CCATCTCATCCCTGCGTGTCTCCGACTCAGAACCTCATTCAGTGGGATTCCTGCTGTCAGT-3' |
| SEQ ID NO:25 | A -BC-LIB_25 | 5'-CCATCTCATCCCTGCGTGTCTCCGACTCAGCCTGAGATACAGTGGGATTCCTGCTGTCAGT-3' |
| SEQ ID NO:26 | A -BC-LIB_26 | 5'-CCATCTCATCCCTGCGTGTCTCCGACTCAGTTACAACCTCAGTGGGATTCCTGCTGTCAGT-3' |
| SEQ ID NO:27 | A -BC-LIB_27 | 5'-CCATCTCATCCCTGCGTGTCTCCGACTCAGAACCATCCGCAGTGGGATTCCTGCTGTCAGT-3' |
| SEQ ID NO:28 | A -BC-LIB_28 | 5'-CCATCTCATCCCTGCGTGTCTCCGACTCAGATCCGGAATCAGTGGGATTCCTGCTGTCAGT-3' |
| SEQ ID NO:29 | A -BC-LIB_29 | 5'-CCATCTCATCCCTGCGTGTCTCCGACTCAGTCGACCACTCAGTGGGATTCCTGCTGTCAGT-3' |
| SEQ ID NO:30 | A -BC-LIB_30 | 5'-CCATCTCATCCCTGCGTGTCTCCGACTCAGCGAGGTTATCAGTGGGATTCCTGCTGTCAGT-3' |

| **SEQ ID NO** | **Primer name** | **Primer sequence** |
|---|---|---|
| SEQ ID NO:31 | A -BC-LIB_31 | 5'-CCATCTCATCCCTGCGTGTCTCCGACTCAGTCCAAGCTGCAGTGGGATTCCTGCTGTCAGT-3' |
| SEQ ID NO:32 | A -BC-LIB_32 | 5'-CCATCTCATCCCTGCGTGTCTCCGACTCAGTCTTACACACAGTGGGATTCCTGCTGTCAGT-3' |
| SEQ ID NO:33 | A -BC-LIB_33 | 5'-CCATCTCATCCCTGCGTGTCTCCGACTCAGTTCTCATTGAACAGTGGGATTCCTGCTGTCAGT-3' |
| SEQ ID NO:34 | A -BC-LIB_34 | 5'-CCATCTCATCCCTGCGTGTCTCCGACTCAGTCGCATCGTTCAGTGGGATTCCTGCTGTCAGT-3' |
| SEQ ID NO:35 | A -BC-LIB_35 | 5'-CCATCTCATCCCTGCGTGTCTCCGACTCAGTAAGCCATTGTCAGTGGGATTCCTGCTGTCAGT-3' |
| SEQ ID NO:36 | A -BC-LIB_36 | 5'-CCATCTCATCCCTGCGTGTCTCCGACTCAGAAGGAATCGTCAGTGGGATTCCTGCTGTCAGT-3' |
| SEQ ID NO:37 | A -BC-LIB_37 | 5'-CCATCTCATCCCTGCGTGTCTCCGACTCAGCTTGAGAATGTCAGTGGGATTCCTGCTGTCAGT-3' |
| SEQ ID NO:38 | A -BC-LIB_38 | 5'-CCATCTCATCCCTGCGTGTCTCCGACTCAGTGGAGGACGGACAGTGGGATTCCTGCTGTCAGT-3' |
| SEQ ID NO:39 | A -BC-LIB_39 | 5'-CCATCTCATCCCTGCGTGTCTCCGACTCAGTAACAATCGGCAGTGGGATTCCTGCTGTCAGT-3' |
| SEQ ID NO:40 | A -BC-LIB_40 | 5'-CCATCTCATCCCTGCGTGTCTCCGACTCAGCTGACATAATCAGTGGGATTCCTGCTGTCAGT-3' |
| SEQ ID NO:41 | A -BC-LIB_41 | 5'-CCATCTCATCCCTGCGTGTCTCCGACTCAGTTCCACTTCGCAGTGGGATTCCTGCTGTCAGT-3' |
| SEQ ID NO:42 | A -BC-LIB_42 | 5'-CCATCTCATCCCTGCGTGTCTCCGACTCAGAGCACGAATCAGTGGGATTCCTGCTGTCAGT-3' |
| SEQ ID NO:43 | A -BC-LIB_43 | 5'-CCATCTCATCCCTGCGTGTCTCCGACTCAGCTTGACACCGCAGTGGGATTCCTGCTGTCAGT-3' |
| SEQ ID NO:44 | A -BC-LIB_44 | 5'-CCATCTCATCCCTGCGTGTCTCCGACTCAGTTGGAGGCCAGCAGTGGGATTCCTGCTGTCAGT-3' |
| SEQ ID NO:45 | A -BC-LIB_45 | 5'-CCATCTCATCCCTGCGTGTCTCCGACTCAGTGGAGCTTCCTCAGTGGGATTCCTGCTGTCAGT-3' |
| SEQ ID NO:46 | A -BC-LIB_46 | 5'-CCATCTCATCCCTGCGTGTCTCCGACTCAGTCAGTCCGAACAGTGGGATTCCTGCTGTCAGT-3' |
| SEQ ID NO:47 | A -BC-LIB_47 | 5'-CCATCTCATCCCTGCGTGTCTCCGACTCAGTAAGGCAACCACAGTGGGATTCCTGCTGTCAGT-3' |
| SEQ ID NO:48 | A -BC-LIB_48 | 5'-CCATCTCATCCCTGCGTGTCTCCGACTCAGTTCTAAGAGACAGTGGGATTCCTGCTGTCAGT-3' |
| SEQ ID NO:49 | A -BC-LIB_49 | 5'-CCATCTCATCCCTGCGTGTCTCCGACTCAGTCCTAACATAACAGTGGGATTCCTGCTGTCAGT-3' |
| SEQ ID NO:50 | A -BC-LIB_50 | 5'-CCATCTCATCCCTGCGTGTCTCCGACTCAGCGGACAATGGCAGTGGGATTCCTGCTGTCAGT-3' |
| SEQID NO:51 | A -BC-LIB_51 | 5'-CCATCTCATCCCTGCGTGTCTCCGACTCAGTTGAGCCTATTCAGTGGGATTCCTGCTGTCAGT-3' |
| SEQ ID NO:52 | A -BC-LIB_52 | 5'-CCATCTCATCCCTGCGTGTCTCCGACTCAGCCGCATGGAACAGTGGGATTCCTGCTGTCAGT-3' |
| SEQ ID NO:53 | A -BC-LIB_53 | 5'-CCATCTCATCCCTGCGTGTCTCCGACTCAGCTGGCAATCCTCAGTGGGATTCCTGCTGTCAGT-3' |
| SEQ ID NO:54 | A -BC-LIB_54 | 5'-CCATCTCATCCCTGCGTGTCTCCGACTCAGCCGGAGAATCGCAGTGGGATTCCTGCTGTCAGT-3' |
| SEQ ID NO:55 | A -BC-LIB_55 | 5'-CCATCTCATCCCTGCGTGTCTCCGACTCAGTCCACCTCCTCAGTGGGATTCCTGCTGTCAGT-3' |
| SEQ ID NO:56 | A -BC-LIB_56 | 5'-CCATCTCATCCCTGCGTGTCTCCGACTCAGCAGCATTAATTCAGTGGGATTCCTGCTGTCAGT-3' |
| SEQ ID NO:57 | A -BC-LIB_57 | 5'-CCATCTCATCCCTGCGTGTCTCCGACTCAGTCTGGCAACGGCAGTGGGATTCCTGCTGTCAGT-3' |
| SEQ ID NO:58 | A -BC-LIB_58 | 5'-CCATCTCATCCCTGCGTGTCTCCGACTCAGTCCTAGAACACAGTGGGATTCCTGCTGTCAGT-3' |
| SEQ ID NO:59 | A -BC-LIB_59 | 5'-CCATCTCATCCCTGCGTGTCTCCGACTCAGTCCTTGATGTTCAGTGGGATTCCTGCTGTCAGT-3' |
| SEQ ID NO:60 | A -BC-LIB_60 | 5'-CCATCTCATCCCTGCGTGTCTCCGACTCAGTCTAGCTCTTCAGTGGGATTCCTGCTGTCAGT-3' |
| SEQ ID NO:61 | A -BC-LIB_61 | 5'-CCATCTCATCCCTGCGTGTCTCCGACTCAGTCACTCGGATCAGTGGGATTCCTGCTGTCAGT-3' |
| SEQ ID NO:62 | A -BC-LIB_62 | 5'-CCATCTCATCCCTGCGTGTCTCCGACTCAGTTCCTGCTTCACAGTGGGATTCCTGCTGTCAGT-3' |
| SEQ ID NO:63 | A -BC-LIB_63 | 5'-CCATCTCATCCCTGCGTGTCTCCGACTCAGCCTTAGAGTTCAGTGGGATTCCTGCTGTCAGT-3' |
| SEQ ID NO:64 | A -BC-LIB_64 | 5'-CCATCTCATCCCTGCGTGTCTCCGACTCAGCTGAGTTCCGACAGTGGGATTCCTGCTGTCAGT-3' |
| SEQ ID NO:65 | A -BC-LIB_65 | 5'-CCATCTCATCCCTGCGTGTCTCCGACTCAGTCCTGGCACATCAGTGGGATTCCTGCTGTCAGT-3' |
| SEQ ID NO:66 | A -BC-LIB_66 | 5'-CCATCTCATCCCTGCGTGTCTCCGACTCAGCCGCAATCATCAGTGGGATTCCTGCTGTCAGT-3' |
| SEQ ID NO:67 | A -BC-LIB_67 | 5'-CCATCTCATCCCTGCGTGTCTCCGACTCAGTTCCTACCAGTCAGTGGGATTCCTGCTGTCAGT-3' |
| SEQ ID NO:68 | A -BC-LIB_68 | 5'-CCATCTCATCCCTGCGTGTCTCCGACTCAGTCAAGAAGTTCAGTGGGATTCCTGCTGTCAGT-3' |
| SEQ ID NO:69 | A -BC-LIB_69 | 5'-CCATCTCATCCCTGCGTGTCTCCGACTCAGTTCAATTGGCAGTGGGATTCCTGCTGTCAGT-3' |
| SEQ ID NO:70 | A -BC-LIB_70 | 5'-CCATCTCATCCCTGCGTGTCTCCGACTCAGCCTACTGGTCAGTGGGATTCCTGCTGTCAGT-3' |
| SEQID NO:71 | A -BC-LIB_71 | 5'-CCATCTCATCCCTGCGTGTCTCCGACTCAGTGAGGCTCCGACAGTGGGATTCCTGCTGTCAGT-3' |
| SEQ ID NO:72 | A -BC-LIB_72 | 5'-CCATCTCATCCCTGCGTGTCTCCGACTCAGCGAAGGCCACACAGTGGGATTCCTGCTGTCAGT-3' |
| SEQ ID NO:73 | A -BC-LIB_73 | 5'-CCATCTCATCCCTGCGTGTCTCCGACTCAGTCTGCCTGTCAGTGGGATTCCTGCTGTCAGT-3' |
| SEQ ID NO:74 | A -BC-LIB_74 | 5'-CCATCTCATCCCTGCGTGTCTCCGACTCAGCGATCGGTTCAGTGGGATTCCTGCTGTCAGT-3' |
| SEQ ID NO:75 | A -BC-LIB_75 | 5'-CCATCTCATCCCTGCGTGTCTCCGACTCAGTCAGGAATACAGTGGGATTCCTGCTGTCAGT-3' |
| SEQ ID NO:76 | A -BC-LIB_76 | 5'-CCATCTCATCCCTGCGTGTCTCCGACTCAGCGGAAGAACCTCAGTGGGATTCCTGCTGTCAGT-3' |
| SEQ ID NO:77 | A -BC-LIB_77 | 5'-CCATCTCATCCCTGCGTGTCTCCGACTCAGCGAAGCGATTCAGTGGGATTCCTGCTGTCAGT-3' |
| SEQ ID NO:78 | A -BC-LIB_78 | 5'-CCATCTCATCCCTGCGTGTCTCCGACTCAGCAGCCAATTCTCAGTGGGATTCCTGCTGTCAGT-3' |
| SEQ ID NO:79 | A -BC-LIB_79 | 5'-CCATCTCATCCCTGCGTGTCTCCGACTCAGCCTGGTTGTCAGTGGGATTCCTGCTGTCAGT-3' |
| SEQ ID NO:80 | A -BC-LIB_80 | 5'-CCATCTCATCCCTGCGTGTCTCCGACTCAGTCGAAGGCAGGCAGTGGGATTCCTGCTGTCAGT-3' |
| SEQ ID NO:81 | A -BC-LIB_81 | 5'-CCATCTCATCCCTGCGTGTCTCCGACTCAGCCTGCCATTCGCAGTGGGATTCCTGCTGTCAGT-3' |
| SEQ ID NO:82 | A -BC-LIB_82 | 5'-CCATCTCATCCCTGCGTGTCTCCGACTCAGTTGGCATCTCAGTGGGATTCCTGCTGTCAGT-3' |
| SEQ ID NO:83 | A -BC-LIB_83 | 5'-CCATCTCATCCCTGCGTGTCTCCGACTCAGCTAGGACATTCAGTGGGATTCCTGCTGTCAGT-3' |
| SEQ ID NO:84 | A -BC-LIB_84 | 5'-CCATCTCATCCCTGCGTGTCTCCGACTCAGCTTCCATAACAGTGGGATTCCTGCTGTCAGT-3' |
| SEQ ID NO:85 | A -BC-LIB_85 | 5'-CCATCTCATCCCTGCGTGTCTCCGACTCAGCCAGCCTCAACAGTGGGATTCCTGCTGTCAGT-3' |
| SEQ ID NO:86 | A -BC-LIB_86 | 5'-CCATCTCATCCCTGCGTGTCTCCGACTCAGCTTGGTTATTCAGTGGGATTCCTGCTGTCAGT-3' |
| SEQ ID NO:87 | A -BC-LIB_87 | 5'-CCATCTCATCCCTGCGTGTCTCCGACTCAGTTGGCTGGACAGTGGGATTCCTGCTGTCAGT-3' |
| SEQ ID NO:88 | A -BC-LIB_88 | 5'-CCATCTCATCCCTGCGTGTCTCCGACTCAGCCGAACACTTCAGTGGGATTCCTGCTGTCAGT-3' |
| SEQ ID NO:89 | A -BC-LIB_89 | 5'-CCATCTCATCCCTGCGTGTCTCCGACTCAGTCCTGAATCTCAGTGGGATTCCTGCTGTCAGT-3' |
| SEQ ID NO:90 | A -BC-LIB_90 | 5'-CCATCTCATCCCTGCGTGTCTCCGACTCAGCTAACCACGGCAGTGGGATTCCTGCTGTCAGT-3' |
| SEQ ID NO:91 | A -BC-LIB_91 | 5'-CCATCTCATCCCTGCGTGTCTCCGACTCAGCGGAAGGATGCAGTGGGATTCCTGCTGTCAGT-3' |
| SEQ ID NO:92 | A -BC-LIB_92 | 5'-CCATCTCATCCCTGCGTGTCTCCGACTCAGCTAGGAACCGCAGTGGGATTCCTGCTGTCAGT-3' |
| SEQ ID NO:93 | A -BC-LIB_93 | 5'-CCATCTCATCCCTGCGTGTCTCCGACTCAGCTTGTCCAATCAGTGGGATTCCTGCTGTCAGT-3' |
| SEQ ID NO:94 | A-BC-LIB_94 | 5'-CCATCTCATCCCTGCGTGTCTCCGACTCAGTCCGACAAGCAGTGGGATTCCTGCTGTCAGT-3' |
| SEQ ID NO:95 | A-BC-LIB_95 | 5'-CCATCTCATCCCTGCGTGTCTCCGACTCAGCGGACAGATCAGTGGGATTCCTGCTGTCAGT-3' |
| SEQ ID NO:96 | A-BC-LIB_96 | 5'-CCATCTCATCCCTGCGTGTCTCCGACTCAGTTAAGCGGTCAGTGGGATTCCTGCTGTCAGT-3' |

b) SEQ ID NO list_second_primer_[PGM/DRS-WGA]

| **SEQ ID NO:** | Primer name | Primer sequence |
|---|---|---|
| SEQ ID NO:97 | P1-LIB | 5'-CCTCTCTATGGGCAGTCGGTGATAGTGGGATTCCTGCTGTCAGT-3' |

c) SEQ ID NO list_first_primer_[PGM/MALBAC]

| **SEQ ID NO** | **primer name** | **Primer sequence** |
|---|---|---|
| SEQ ID NO:98 | A -BC-MALBAC_1 | 5'-CCATCTCATCCCTGCGTGTCTCCGACTCAGCTAAGGTAACGTGAGTGATGGTTGAGGTAGTGTGGAG-3' |
| SEQ ID NO:99 | A -BC-MALBAC_2 | 5'-CCATCTCATCCCTGCGTGTCTCCGACTCAGTAAGGAGAACGTGAGTGATGGTTGAGGTAGTGTGGAG-3' |
| SEQ ID NO:100 | A -BC-MALBAC_3 | 5'-CCATCTCATCCCTGCGTGTCTCCGACTCAGAAGAGGATTCGTGAGTGATGGTTGAGGTAGTGTGGAG-3' |
| SEQ ID NO:101 | A -BC-MALBAC_4 | 5'-CCATCTCATCCCTGCGTGTCTCCGACTCAGTACCAAGATCGTGAGTGATGGTTGAGGTAGTGTGGAG-3' |
| SEQ ID NO:102 | A -BC-MALBAC_5 | 5'-CCATCTCATCCCTGCGTGTCTCCGACTCAGCAGAAGGAACGTGAGTGATGGTTGAGGTAGTGTGGAG-3' |
| SEQ ID NO:103 | A -BC-MALBAC_6 | 5'-CCATCTCATCCCTGCGTGTCTCCGACTCAGCTGCAAGTTCGTGAGTGATGGTTGAGGTAGTGTGGAG-3' |
| SEQ ID NO:104 | A -BC-MALBAC_7 | 5'-CCATCTCATCCCTGCGTGTCTCCGACTCAGTTCGTGATTCGTGAGTGATGGTTGAGGTAGTGTGGAG-3' |
| SEQ ID NO:105 | A -BC-MALBAC_8 | 5'-CCATCTCATCCCTGCGTGTCTCCGACTCAGTTCCGATAACGTGAGTGATGGTTGAGGTAGTGTGGAG-3' |
| SEQ ID NO:106 | A -BC-MALBAC_9 | 5'-CCATCTCATCCCTGCGTGTCTCCGACTCAGTGAGCGGAACGTGAGTGATGGTTGAGGTAGTGTGGAG-3' |
| SEQ ID NO:107 | A -BC-MALBAC_10 | 5'-CCATCTCATCCCTGCGTGTCTCCGACTCAGCTGACCGAACGTGAGTGATGGTTGAGGTAGTGTGGAG-3' |
| SEQ ID NO:108 | A -BC-MALBAC_11 | 5'-CCATCTCATCCCTGCGTGTCTCCGACTCAGTCCTCGAATCGTGAGTGATGGTTGAGGTAGTGTGGAG-3' |
| SEQ ID NO:109 | A -BC-MALBAC_12 | 5'-CCATCTCATCCCTGCGTGTCTCCGACTCAGTAGGTGGTTCGTGAGTGATGGTTGAGGTAGTGTGGAG-3' |
| SEQ ID NO:110 | A -BC-MALBAC_13 | 5'-CCATCTCATCCCTGCGTGTCTCCGACTCAGTCTAACGGACGTGAGTGATGGTTGAGGTAGTGTGGAG-3' |
| SEQ ID NO:111 | A -BC-MALBAC_14 | 5'-CCATCTCATCCCTGCGTGTCTCCGACTCAGTTGGAGTGTCGTGAGTGATGGTTGAGGTAGTGTGGAG-3' |
| SEQ ID NO:112 | A -BC-MALBAC_15 | 5'-CCATCTCATCCCTGCGTGTCTCCGACTCAGTCTAGAGGTCGTGAGTGATGGTTGAGGTAGTGTGGAG-3' |
| SEQ ID NO:113 | A -BC-MALBAC_16 | 5'-CCATCTCATCCCTGCGTGTCTCCGACTCAGTCTGGATGACGTGAGTGATGGTTGAGGTAGTGTGGAG-3' |
| SEQ ID NO:114 | A -BC-MALBAC_17 | 5'-CCATCTCATCCCTGCGTGTCTCCGACTCAGTCTATTCGTCGTGAGTGATGGTTGAGGTAGTGTGGAG-3' |
| SEQ ID NO:115 | A -BC-MALBAC_18 | 5'-CCATCTCATCCCTGCGTGTCTCCGACTCAGAGGCAATTGCGTGAGTGATGGTTGAGGTAGTGTGGAG-3' |
| SEQ ID NO:116 | A -BC-MALBAC_19 | 5'-CCATCTCATCCCTGCGTGTCTCCGACTCAGTTAGTCGGACGTGAGTGATGGTTGAGGTAGTGTGGAG-3' |
| SEQ ID NO:117 | A -BC-MALBAC_20 | 5'-CCATCTCATCCCTGCGTGTCTCCGACTCAGCAGATCCATCGTGAGTGATGGTTGAGGTAGTGTGGAG-3' |
| SEQ ID NO:118 | A -BC-MALBAC_21 | 5'-CCATCTCATCCCTGCGTGTCTCCGACTCAGTCGCAATTACGTGAGTGATGGTTGAGGTAGTGTGGAG-3' |
| SEQ ID NO:119 | A-BC-MALBAC_22 | 5'-CCATCTCATCCCTGCGTGTCTCCGACTCAGTTCGAGACGCGTGAGTGATGGTTGAGGTAGTGTGGAG-3' |
| SEQ ID NO:120 | A-BC-MALBAC_23 | 5'-CCATCTCATCCCTGCGTGTCTCCGACTCAGTGCCACGAACGTGAGTGATGGTTGAGGTAGTGTGGAG-3' |
| SEQ ID NO:121 | A-BC-MALBAC_24 | 5'-CCATCTCATCCCTGCGTGTCTCCGACTCAGAACCTCATTCGTGAGTGATGGTTGAGGTAGTGTGGAG-3' |
| SEQ ID NO:122 | A-BC-MALBAC_25 | 5'-CCATCTCATCCCTGCGTGTCTCCGACTCAGCCTGAGATACGTGAGTGATGGTTGAGGTAGTGTGGAG-3' |
| SEQ ID NO:123 | A-BC-MALBAC_26 | 5'-CCATCTCATCCCTGCGTGTCTCCGACTCAGTTACAACCTCGTGAGTGATGGTTGAGGTAGTGTGGAG-3' |
| SEQ ID NO:124 | A-BC-MALBAC_27 | 5'-CCATCTCATCCCTGCGTGTCTCCGACTCAGAACCATCCGCGTGAGTGATGGTTGAGGTAGTGTGGAG-3' |
| SEQ ID NO:125 | A-BC-MALBAC_28 | 5'-CCATCTCATCCCTGCGTGTCTCCGACTCAGATCCGGAATCGTGAGTGATGGTTGAGGTAGTGTGGAG-3' |
| SEQ ID NO:126 | A-BC-MALBAC_29 | 5'-CCATCTCATCCCTGCGTGTCTCCGACTCAGTCGACCACTCGTGAGTGATGGTTGAGGTAGTGTGGAG-3' |
| SEQ ID NO:127 | A-BC-MALBAC_30 | 5'-CCATCTCATCCCTGCGTGTCTCCGACTCAGCGAGGTTATCGTGAGTGATGGTTGAGGTAGTGTGGAG-3' |
| SEQ ID NO:128 | A-BC-MALBAC_31 | 5'-CCATCTCATCCCTGCGTGTCTCCGACTCAGTCCAAGCTGCGTGAGTGATGGTTGAGGTAGTGTGGAG-3' |
| SEQ ID NO:129 | A-BC-MALBAC_32 | 5'-CCATCTCATCCCTGCGTGTCTCCGACTCAGTCTTACACACGTGAGTGATGGTTGAGGTAGTGTGGAG-3' |
| SEQ ID NO:130 | A-BC-MALBAC_33 | 5'-CCATCTCATCCCTGCGTGTCTCCGACTCAGTTCTCATTGAACGTGAGTGATGGTTGAGGTAGTGTGGAG-3' |
| SEQ ID NO:131 | A-BC-MALBAC_34 | 5'-CCATCTCATCCCTGCGTGTCTCCGACTCAGTCGCATCGTTCGTGAGTGATGGTTGAGGTAGTGTGGAG-3' |
| SEQ ID NO:132 | A-BC-MALBAC_35 | 5'-CCATCTCATCCCTGCGTGTCTCCGACTCAGTAAGCCATTGTCGTGAGTGATGGTTGAGGTAGTGTGGAG-3' |
| SEQ ID NO:133 | A-BC-MALBAC_36 | 5'-CCATCTCATCCCTGCGTGTCTCCGACTCAGAAGGAATCGTCGTGAGTGATGGTTGAGGTAGTGTGGAG-3' |
| SEQ ID NO:134 | A-BC-MALBAC_37 | 5'-CCATCTCATCCCTGCGTGTCTCCGACTCAGCTTGAGAATGTCGTGAGTGATGGTTGAGGTAGTGTGGAG-3' |
| SEQ ID NO:135 | A-BC-MALBAC_38 | 5'-CCATCTCATCCCTGCGTGTCTCCGACTCAGTGGAGGACGGACGTGAGTGATGGTTGAGGTAGTGTGGAG-3' |
| SEQ ID NO:136 | A-BC-MALBAC_39 | 5'-CCATCTCATCCCTGCGTGTCTCCGACTCAGTAACAATCGGCGTGAGTGATGGTTGAGGTAGTGTGGAG-3' |
| SEQ ID NO:137 | A-BC-MALBAC_40 | 5'-CCATCTCATCCCTGCGTGTCTCCGACTCAGCTGACATAATCGTGAGTGATGGTTGAGGTAGTGTGGAG-3' |
| SEQ ID NO:138 | A-BC-MALBAC_41 | 5'-CCATCTCATCCCTGCGTGTCTCCGACTCAGTTCCACTTCGCGTGAGTGATGGTTGAGGTAGTGTGGAG-3' |
| SEQ ID NO:139 | A-BC-MALBAC_42 | 5'-CCATCTCATCCCTGCGTGTCTCCGACTCAGAGCACGAATCGTGAGTGATGGTTGAGGTAGTGTGGAG-3' |
| SEQ ID NO:140 | A-BC-MALBAC_43 | 5'-CCATCTCATCCCTGCGTGTCTCCGACTCAGCTTGACACCGCGTGAGTGATGGTTGAGGTAGTGTGGAG-3' |
| SEQ ID NO:141 | A-BC-MALBAC_44 | 5'-CCATCTCATCCCTGCGTGTCTCCGACTCAGTTGGAGGCCAGCGTGAGTGATGGTTGAGGTAGTGTGGAG-3' |
| SEQ ID NO:142 | A-BC-MALBAC_45 | 5'-CCATCTCATCCCTGCGTGTCTCCGACTCAGTGGAGCTΓCCTCGTGAGTGATGGTTGAGGTAGTGTGGAG-3' |
| SEQ ID NO:143 | A-BC-MALBAC_46 | 5'-CCATCTCATCCCTGCGTGTCTCCGACTCAGTCAGTCCGAACGTGAGTGATGGTTGAGGTAGTGTGGAG-3' |
| SEQ ID NO:144 | A-BC-MALBAC_47 | 5'-CCATCTCATCCCTGCGTGTCTCCGACTCAGTAAGGCAACCACGTGAGTGATGGTTGAGGTAGTGTGGAG-3' |
| SEQ ID NO:145 | A-BC-MALBAC_48 | 5'-CCATCTCATCCCTGCGTGTCTCCGACTCAGTTCTAAGAGACGTGAGTGATGGTTGAGGTAGTGTGGAG-3' |
| SEQ ID NO:146 | A-BC-MALBAC_49 | 5'-CCATCTCATCCCTGCGTGTCTCCGACTCAGTCCTAACATAACGTGAGTGATGGTTGAGGTAGTGTGGAG-3' |
| SEQ ID NO:147 | A-BC-MALBAC_50 | 5'-CCATCTCATCCCTGCGTGTCTCCGACTCAGCGGACAATGGCGTGAGTGATGGTTGAGGTAGTGTGGAG-3' |
| SEQ ID NO:148 | A-BC-MALBAC_51 | 5'-CCATCTCATCCCTGCGTGTCTCCGACTCAGTTGAGCCTATTCGTGAGTGATGGTTGAGGTAGTGTGGAG-3' |
| SEQ ID NO:149 | A-BC-MALBAC_52 | 5'-CCATCTCATCCCTGCGTGTCTCCGACTCAGCCGCATGGAACGTGAGTGATGGTTGAGGTAGTGTGGAG-3' |
| SEQ ID NO:150 | A-BC-MALBAC_53 | 5'-CCATCTCATCCCTGCGTGTCTCCGACTCAGCTGGCAATCCTCGTGAGTGATGGTTGAGGTAGTGTGGAG-3' |
| SEQ ID NO:151 | A-BC-MALBAC_54 | 5'-CCATCTCATCCCTGCGTGTCTCCGACTCAGCCGGAGAATCGCGTGAGTGATGGTTGAGGTAGTGTGGAG-3' |
| SEQ ID NO:152 | A-BC-MALBAC_55 | 5'-CCATCTCATCCCTGCGTGTCTCCGACTCAGTCCACCTCCTCGTGAGTGATGGTTGAGGTAGTGTGGAG-3' |
| SEQ ID NO:153 | A-BC-MALBAC_56 | 5'-CCATCTCATCCCTGCGTGTCTCCGACTCAGCAGCATTAATTCGTGAGTGATGGTTGAGGTAGTGTGGAG-3' |
| SEQ ID NO:154 | A-BC-MALBAC_57 | 5'-CCATCTCATCCCTGCGTGTCTCCGACTCAGTCTGGCAACGGCGTGAGTGATGGTTGAGGTAGTGTGGAG-3' |
| SEQ ID NO:155 | A-BC-MALBAC_58 | 5'-CCATCTCATCCCTGCGTGTCTCCGACTCAGTCCTAGAACACGTGAGTGATGGTTGAGGTAGTGTGGAG-3' |
| SEQ ID NO:156 | A-BC-MALBAC_59 | 5'-CCATCTCATCCCTGCGTGTCTCCGACTCAGTCCTTGATGTTCGTGAGTGATGGTTGAGGTAGTGTGGAG-3' |
| SEQ ID NO:157 | A-BC-MALBAC_60 | 5'-CCATCTCATCCCTGCGTGTCTCCGACrCAGTCTAGCTCTTCGTGAGTGATGGTTGAGGTAGTGTGGAG-3' |
| SEQ ID NO:158 | A-BC-MALBAC_61 | 5'-CCATCTCATCCCTGCGTGTCTCCGACTCAGTCACTCGGATCGTGAGTGATGGTTGAGGTAGTGTGGAG-3' |
| SEQ ID NO:159 | A-BC-MALBAC_62 | 5'-CCATCTCATCCCTGCGTGTCTCCGACTCAGTTCCTGCTTCACGTGAGTGATGGTTGAGGTAGTGTGGAG-3' |
| SEQ ID NO:160 | A-BC-MALBAC_63 | 5'-CCATCTCATCCCTGCGTGTCTCCGACTCAGCCTTAGAGTTCGTGAGTGATGGTTGAGGTAGTGTGGAG-3' |
| SEQ ID NO:161 | A-BC-MALBAC_64 | 5'-CCATCTCATCCCTGCGTGTCTCCGACTCAGCTGAGTTCCGACGTGAGTGATGGTTGAGGTAGTGTGGAG-3' |
| SEQ ID NO:162 | A-BC-MALBAC_65 | 5'-CCATCTCATCCCTGCGTGTCTCCGACTCAGTCCTGGCACATCGTGAGTGATGGTTGAGGTAGTGTGGAG-3' |
| SEQ ID NO:163 | A-BC-MALBAC_66 | 5'-CCATCTCATCCCTGCGTGTCTCCGACTCAGCCGCAATCATCGTGAGTGATGGTTGAGGTAGTGTGGAG-3' |
| SEQ ID NO:164 | A-BC-MALBAC_67 | 5'-CCATCTCATCCCTGCGTGTCTCCGACTCAGTTCCTACCAGTCGTGAGTGATGGTTGAGGTAGTGTGGAG-3' |
| SEQ ID NO:165 | A-BC-MALBAC_68 | 5'-CCATCTCATCCCTGCGTGTCTCCGACTCAGTCAAGAAGTTCGTGAGTGATGGTTGAGGTAGTGTGGAG-3' |
| SEQ ID NO:166 | A-BC-MALBAC_69 | 5'-CCATCTCATCCCTGCGTGTCTCCGACTCAGTTCAATTGGCGTGAGTGATGGTTGAGGTAGTGTGGAG-3' |
| SEQ ID NO:167 | A-BC-MALBAC_70 | 5'-CCATCTCATCCCTGCGTGTCTCCGACTCAGCCTACTGGTCGTGAGTGATGGTTGAGGTAGTGTGGAG-3' |
| SEQ ID NO:168 | A-BC-MALBAC_71 | 5'-CCATCTCATCCCTGCGTGTCTCCGACTCAGTGAGGCTCCGACGTGAGTGATGGTTGAGGTAGTGTGGAG-3' |
| SEQ ID NO:169 | A-BC-MALBAC_72 | 5'-CCATCTCATCCCTGCGTGTCTCCGACTCAGCGAAGGCCACACGTGAGTGATGGTTGAGGTAGTGTGGAG-3' |
| SEQ ID NO:170 | A-BC-MALBAC_73 | 5'-CCATCTCATCCCTGCGTGTCTCCGACTCAGTCTGCCTGTCGTGAGTGATGGTTGAGGTAGTGTGGAG-3' |
| SEQ ID NO:171 | A-BC-MALBAC_74 | 5'-CCATCTCATCCCTGCGTGTCTCCGACTCAGCGATCGGTTCGTGAGTGATGGTTGAGGTAGTGTGGAG-3' |
| SEQ ID NO:172 | A-BC-MALBAC_75 | 5'-CCATCTCATCCCTGCGTGTCTCCGACTCAGTCAGGAATACGTGAGTGATGGTTGAGGTAGTGTGGAG-3' |
| SEQ ID NO:173 | A-BC-MALBAC_76 | 5'-CCATCTCATCCCTGCGTGTCTCCGACTCAGCGGAAGAACCTCGTGAGTGATGGTTGAGGTAGTGTGGAG-3' |
| SEQ ID NO:174 | A-BC-MALBAC_77 | 5'-CCATCTCATCCCTGCGTGTCTCCGACTCAGCGAAGCGATTCGTGAGTGATGGTTGAGGTAGTGTGGAG-3' |
| SEQ ID NO:175 | A-BC-MALBAC_78 | 5'-CCATCTCATCCCTGCGTGTCTCCGACTCAGCAGCCAATTCTCGTGAGTGATGGTTGAGGTAGTGTGGAG-3' |
| SEQ ID NO:176 | A-BC-MALBAC_79 | 5'-CCATCTCATCCCTGCGTGTCTCCGACTCAGCCTGGTTGTCGTGAGTGATGGTTGAGGTAGTGTGGAG-3' |
| SEQ ID NO:177 | A-BC-MALBAC_80 | 5'-CCATCTCATCCCTGCGTGTCTCCGACTCAGTCGAAGGCAGGCGTGAGTGATGGTTGAGGTAGTGTGGAG-3' |
| SEQ ID NO:178 | A-BC-MALBAC_81 | 5'-CCATCTCATCCCTGCGTGTCTCCGACTCAGCCTGCCATTCGCGTGAGTGATGGTTGAGGTAGTGTGGAG-3' |
| SEQ ID NO:179 | A-BC-MALBAC_82 | 5'-CCATCTCATCCCTGCGTGTCTCCGACTCAGTTGGCATCTCGTGAGTGATGGTTGAGGTAGTGTGGAG-3' |
| SEQ ID NO:180 | A-BC-MALBAC_83 | 5'-CCATCTCATCCCTGCGTGTCTCCGACTCAGCTAGGACATTCGTGAGTGATGGTTGAGGTAGTGTGGAG-3' |
| SEQ ID NO:181 | A-BC-MALBAC_84 | 5'-CCATCTCATCCCTGCGTGTCTCCGACTCAGCTTCCATAACGTGAGTGATGGTTGAGGTAGTGTGGAG-3' |
| SEQ ID NO:182 | A-BC-MALBAC_85 | 5'-CCATCTCATCCCTGCGTGTCTCCGACTCAGCCAGCCTCAACGTGAGTGATGGTTGAGGTAGTGTGGAG-3' |
| SEQ ID NO:183 | A-BC-MALBAC_86 | 5'-CCATCTCATCCCTGCGTGTCTCCGACTCAGCTTGGTTATTCGTGAGTGATGGTTGAGGTAGTGTGGAG-3' |
| SEQ ID NO:184 | A-BC-MALBAC_87 | 5'-CCATCTCATCCCTGCGTGTCTCCGACTCAGTTGGCTGGACGTGAGTGATGGTTGAGGTAGTGTGGAG-3' |
| SEQ ID NO:185 | A-BC-MALBAC_88 | 5'-CCATCTCATCCCTGCGTGTCTCCGACTCAGCCGAACACTTCGTGAGTGATGGTTGAGGTAGTGTGGAG-3' |
| SEQ ID NO:186 | A-BC-MALBAC_89 | 5'-CCATCTCATCCCTGCGTGTCTCCGACTCAGTCCTGAATCTCGTGAGTGATGGTTGAGGTAGTGTGGAG-3' |
| SEQ ID NO:187 | A-BC-MALBAC_90 | 5'-CCATCTCATCCCTGCGTGTCTCCGACTCAGCTAACCACGGCGTGAGTGATGGTTGAGGTAGTGTGGAG-3' |
| SEQ ID NO:188 | A-BC-MALBAC_91 | 5'-CCATCTCATCCCTGCGTGTCTCCGACTCAGCGGAAGGATGCGTGAGTGATGGTTGAGGTAGTGTGGAG-3' |
| SEQ ID NO:189 | A-BC-MALBAC_92 | 5'-CCATCTCATCCCTGCGTGTCTCCGACTCAGCTAGGAACCGCGTGAGTGATGGTTGAGGTAGTGTGGAG-3' |
| SEQ ID NO:190 | A-BC-MALBAC_93 | 5'-CCATCTCATCCCTGCGTGTCTCCGACTCAGCTTGTCCAATCGTGAGTGATGGTTGAGGTAGTGTGGAG-3' |
| SEQ ID NO:191 | A-BC-MALBAC_94 | 5'-CCATCTCATCCCTGCGTGTCTCCGACTCAGTCCGACAAGCGTGAGTGATGGTTGAGGTAGTGTGGAG-3' |
| SEQ ID NO:192 | A-BC-MALBAC_95 | 5'-CCATCTCATCCCTGCGTGTCTCCGACTCAGCGGACAGATCGTGAGTGATGGTTGAGGTAGTGTGGAG-3' |
| SEQ ID NO:193 | A-BC-MALBAC_96 | 5'-CCATCTCATCCCTGCGTGTCTCCGACTCAGTTAAGCGGTCGTGAGTGATGGTTGAGGTAGTGTGGAG-3' |

d) SEQ ID NO list_second_primer_[PGM/MALBAC]

| **SEQ ID -NO** | **primer name** | **Primer sequence** |
|---|---|---|
| SEQ ID NO:194 | P1-MALBAC | 5'-CCTCTCTATGGGCAGTCGGTGATGTGAGTGATGGTTGAGGTAGTGTGGA G-3' |

### Example 2:

### Protocol for LPWGS on Ion Torrent Proton following DRS-WGA

### 1. Deterministic-Restriction Site Whole genome amplification (DRS-WGA)

Single cell DNA was amplified using the *Ampli1^{™}* WGA Kit (Menarini Silicon Biosystems) according to the manufacturer's instructions, as detailed in previous example.

### 2. Double strand DNA synthesis

Five µL of WGA-amplified DNA were converted into double strand DNA (dsDNA) using the *Ampli1^{™}* ReAmp/ds Kit, according to the manufacturing protocol. This process ensures the conversion of single strand DNA (ssDNA) molecules into dsDNA molecules.

### 3. Purification of dsDNA products

Six µL of dsDNA synthesis products were diluted adding 44 µL of Nuclease-Free Water and purified by Agencourt AMPure XP beads (Beckman Coulter) in order to remove unbound oligonucleotides and excess nucleotides, salts and enzymes. The beads-based DNA purification was performed according to the following protocol: 75 µL (ratio: 1.5X of sample volume) of Agencourt AMPure XP beads were added to each 50 µl sample and mixed by vortexing. Mixed reactions were then incubated off-magnet for 15 minutes at room temperature (RT), after which they were placed on a magnetic plate until the solution clears and a pellet is formed (≈ 5 minutes). Then, the supernatant was removed and discarded without disturbing the pellet (approximately 5 µl may be left behind), the beads were washed twice with 150 µL of freshly made 70% EtOH leaving the tube on the magnetic plate. After removing any residual ethanol solution from the bottom of the tube the beads pellet was briefly air-dry. 22 µL of 10mM Tris Ultrapure, pH 8.0, and 0.1mM EDTA (Low TE) buffer were added and the mixed reaction was incubated at room temperature for 2 minutes off the magnetic plate, followed by 5 minutes incubation on magnetic plate. 20 µL of the eluate was transferred into a new tube.

Otherwise, an alternative step 3 (described below), was used in order to produce a uniform distribution of fragments around an average size.

### Alternative step 3) Double purification of dsDNA products

SPRIselect is a SPRI-based chemistry that speeds and simplifies nucleic acid size selection for fragment library preparation for Next Generation sequencing. This step could be performed alternatively to the step 3. Six µL of dsDNA synthesis products were diluted adding 44 µL of Nuclease-Free Water and purified by SPRIselect beads (Beckman Coulter) in order to remove unbound oligonucleotides and excess nucleotides, salts and enzymes and in order to produce a uniform distribution of fragments around an average size. The SPRI-based DNA purification was performed according to the following protocol: 37.5 µL (ratio: 0.75X of sample volume) of SPRIselect beads were added to each 50 µl sample and mixed by vortexing. Mixed reactions were then incubated off-magnet for 15 minutes at RT, after which they were placed on a magnetic plate until the solution clears and a pellet is formed (≈ 5 minutes). Then, the supernatant was recovered and transferred into a new tube. The second round of purification was performed adding 37.5 µL of SPRIselect beads to the supernatant and mixed by vortexing. Mixed reactions were then incubated off-magnet for 15 minutes at RT, after which they were placed on a magnetic plate until the solution clears and a pellet is formed (≈ 5 minutes). Then, the supernatant was removed and discarded without disturbing the pellet (approximately 5 µl may be left behind), the beads were washed twice with 150 µL of freshly made 80% EtOH leaving the tube on the magnetic plate. After removing any residual ethanol solution from the bottom of the tube the beads pellet were briefly air-dry. 22 µL of Low TE buffer were added and the mixed reaction was incubate at room temperature for 2 minutes off the magnet, followed by 5 minutes incubation on magnetic plate. 20 µL of the eluate were transferred into a new tube.

### 4. Barcoded Re-amplification

Barcoded re-amplification was performed in a volume of 50 µl using *Ampli1^{™}* PCR Kit (Menarini Silicon Biosystems). Each PCR reaction was composed as following: 5 µl *Ampli1^{™}* PCR Reaction Buffer (10×), 1 µl of 25 µM of one primer of SEQ ID NO:1 to SEQ ID NO:96
[1] **(5'-CCATCTCATCCCTGCGTGTCTCCGACTCAG[BC]AGTGGGATTCCTGCTGTCAGT-3')**
   where [BC] = Barcode sequence, 1 µl of 25 µM of the primer of SEQ ID NO:97
**[2] (5'-CCTCTCTATGGGCAGTCGGTGATAGTGGGATTCCTGCTGTCAGT-3')**
   1.75 µl *Ampli1^{™}* PCR dNTPs (10 mM), 1.25 µl BSA, 0.5 Ampli1*^{™}* PCR Tag Polymerase (FAST start), 37.5 µl of *Ampli1^{™}* water and 2 µl of the ds-purified DNA. These are the same primers used for Ion Torrent PGM, reported in the corresponding Table of NGS re-amplification primers for Ion Torrent library (DRS WGA for PGM/Proton) displayed above.

Applied Biosystems^{®} 2720 Thermal Cycler was set as follows: 95°C for 4 min, 11 cycles of 95°C for 30 seconds, 60°C for 30 seconds, 72°C for 15 seconds, then a final extension at 72° C for 30 seconds.

### 5. Purification of barcoded re-amplified dsDNA products

Barcoded re-amplified dsDNA products were purified with a ratio 1.5X (75 µl) AMPure XP beads, according to the step 3 described above, and eluted in 35 µl of Low TE buffer. The eluate was transferred to new tube and subsequently quantified by dsDNA HS Assay on the Qubit^{®} 2.0 Fluorometer in order to obtain a final equimolar samples pool. The equimolar pool was created by combining the same amount of each library with different A-LIB-BC-X adapters, producing the final pool with the concentration of 34 ng/µL in a final volume of 42 µL.

### 6. Size selection

E-Gel^{®} SizeSelect^{™} system in combination with Size Select 2% precast agarose gel (Invitrogen) was used for the size selection of fragments of interest, according to the manufacturer's instructions.

Twenty µL of the final pool were loaded on two lanes of an E-gel and using a size standard (50bp DNA Ladder, Invitrogen), a section range between 300 to 400 bp has been selected from the gel. Following size selection, the clean-up was performed with 1.8X (90 µl) AMPure XP beads according to the step 3 described above. Final library was eluted in 30 µl of Low TE buffer.

### 7. Ion Torrent Proton sequencing

The equimolar pool, after the purification step, was qualified by Agilent DNA High Sensitivity Kit on the 2100 Bioanalyzer^{®} (Agilent) and quantified using Qubit^{®} dsDNA HS Assay Kit. Finally, the equimolar pool was diluted to 100 pM final concentration.

Template preparation was performed according to the Ion PI^{™} Hi-Q^{™} Chef user guide. The Ion Chef^{™} System provides automated, high-throughput template preparation and chip loading for use with the Ion Proton^{™} Sequencer. The Ion Proton^{™} Sequencer performs automated high-throughput sequencing of libraries loaded onto Ion PI^{™} Chip using the Ion Proton^{™} Hi-Q^{™} Sequencing Kit (Life Technologies). Finally, the sequenced fragments were assigned to specific samples based on their unique barcode.

### Example 3:

### Protocols for Low Pass Whole Genome Sequencing on Illumina MiSeq Protocol 1

### • Deterministic-Restriction Site Whole genome amplification (DRS-WGA) :

Single cell DNA was amplified using the *Ampli1*^{™} WGA Kit (Silicon Biosystems) according to the manufacturer's instructions. Five µL of WGA-amplified DNA were diluted by the addition of 5 µL of Nuclease-Free Water and purified using Agencourt AMPure XP system (ratio 1.8x). The DNA was eluted in 12.5 µL and quantified by dsDNA HS Assay on the Qubit^{®} 2.0 Fluorometer.

### • Barcoded Re-amplification

Barcoded re-amplification was performed as shown schematically in figure 4, in a volume of 50 µl using *Ampli1^{™}* PCR Kit (Menarini Silicon Biosystems). Each PCR reaction was composed as following: 5 µl *Ampli1^{™}* PCR Reaction Buffer (10X), 1 µl of one primer of SEQ ID NO:195 to SEQ ID NO:202 (25 µM), 1 µl of one primer of SEQ ID NO:203 to SEQ ID NO:214 primer(25 µM), 1.75 µl *Ampli1*^{™} PCR dNTPs (10 mM), 1.25 µl BSA, 0.5 *Ampli1^{™}* PCR Taq Polymerase, 25 ng of the WGA-purified DNA and *Ampli1^{™}* water to reach a final volume of 50 µl.

Applied Biosystems^{®} 2720 Thermal Cycler was set as follows: 95°C for 4 minutes, 1 cycle of 95°C for 30 seconds, 60°C for 30 seconds, 72°C for 2 minutes, 10 cycles of 95°C for 30 seconds, 60°C for 30 seconds, 72°C for 2 minutes (extended by 20 seconds/cycle) and final extension at 72° C for 7 minutes.

Barcoded re-amplified WGA products (containing Illumina sequencing adapter sequences taken from the list SEQ_IDs_ILL_PR1) were then qualified by Agilent DNA 7500 Kit on the 2100 Bioanalyzer^{®} and quantified by Qubit^{®} 2.0 Fluorometer.

### • Size selection

Libraries were then combined at equimolar concentration and the resulting pool, with a concentration of 28.6 ng/µL and a final volume 100 µL, was size-selected by double-purification with SPRI beads. Briefly, SPRI beads were diluted 1:2 with PCR grade water. 160 µL of diluted SPRI beads were added to the 100 µl of pool. After incubation, 25 µL of supernatant were transferred to a new vial and 30 µL of diluted SPRI beads were added. The DNA was eluted in 20 µL of low TE. Fragment size was verified by 2100 Bioanalyzer High Sensitivity Chip (Agilent Technologies) and library quantification was performed by qPCR using the Kapa Library quantification kit.

### • MiSeq sequencing

4 nM of the size-selected pool was denatured 5 minutes with NaOH (NaOH final concentration equal to 0.1N). Denatured sample was then diluted with HT1 to obtain a 20 pM denatured library in 1 mM NaOH. 570 µL of 20 pM denatured library and 30 µl of 20 pM denatured PhiX control were loaded on a MiSeq (Illumina).

Single end reads of 150 bases were generated using the v3 chemistry of the Illumina MiSeq.
SEQ ID NO list_first_primer_[ILLUMINA/DRS-WGA] protocol1

The following table illustrate the structure of the primers DRS-WGA compatible for Illumina platform (sequences in 5' → 3' direction, 5' and 3' omitted):

**TABLE 3**

| | P5/primerindex2 | i5 | primer read1 | atailing | spacer | LIB |
|---|---|---|---|---|---|---|
| LP_DI_D501 | AATGATACGGCGACCACCGAGATCTACAC | TATAGCCT | ACACTCTTTCCCTACACGACGCTCTTCCGATCT | | | AGTGGGATTCCTGCTGTCAGT |
| LP_DI_D502 | AATGATACGGCGACCACCGAGATCTACAC | ATAGAGGC | ACACTCTTTCCCTACACGACGCTCTTCCGATCT | | T | AGTGGGATTCCTGCTGTCAGT |
| LP_DI_D503 | AATGATACGGCGACCACCGAGATCTACAC | CCTATCCT | ACACTCTTTCCCTACACGACGCTCTTCCGATCT | | CT | AGTGGGATTCCTGCTGTCAGT |
| LP_DI_D504 | AATGATACGGCGACCACCGAGATCTACAC | GGCTCTGA | ACACTCTTTCCCTACACGACGCTCTTCCGATCT | | GCC | AGTGGGATTCCTGCTGTCAGT |
| LP_DI_D505 | AATGATACGGCGACCACCGAGATCTACAC | AGGCGAAG | ACACTCTTTCCCTACACGACGCTCTTCCGATCT | | GTCCC | AGTGGGATTCCTGCTGTCAGT |
| LP_DI_D506 | AATGATACGGCGACCACCGAGATCTACAC | TAATCTTA | ACACTCTTTCCCTACACGACGCTCTTCCGATCT | | TCAC | AGTGGGATTCCTGCTGTCAGT |
| LP_DI_D507 | AATGATACGGCGACCACCGAGATCTACAC | CAGGACGT | ACACTCTTTCCCTACACGACGCTCTTCCGATCT | | | AGTGGGATTCCTGCTGTCAGT |
| LP_DI_D508 | AATGATACGGCGACCACCGAGATCTACAC | GTACTGAC | ACACTCTTTCCCTACACGACGCTCTTCCGATCT | | C | AGTGGGATTCCTGCTGTCAGT |
| | | | | | | |

| | P7rc | i7rc | primer read2 | | | LIB |
|---|---|---|---|---|---|---|
| LP_DI_D701 | CAAGCAGAAGACGGCATACGAGAT | CGAGTAAT | GTGACTGGAGTTCAGACGTGTGCTCTTCCGATC | T | | AGTGGGATTCCTGCTGTCAGT |
| LP_DI_D702 | CAAGCAGAAGACGGCATACGAGAT | TCTCCGGA | GTGACTGGAGTTCAGACGTGTGCTCTTCCGATC | T | T | AGTGGGATTCCTGCTGTCAGT |
| LP_DI_D703 | CAAGCAGAAGACGGCATACGAGAT | AATGAGCG | GTGACTGGAGTTCAGACGTGTGCTCTTCCGATC | T | CT | AGTGGGATTCCTGCTGTCAGT |
| LP_DI_D704 | CAAGCAGAAGACGGCATACGAGAT | GGAATCTC | GTGACTGGAGTTCAGACGTGTGCTCTTCCGATC | T | GCC | AGTGGGATTCCTGCTGTCAGT |
| LP_DI_D705 | CAAGCAGAAGACGGCATACGAGAT | TTCTGAAT | GTGACTGGAGTTCAGACGTGTGCTCTTCCGATC | T | GTCCC | AGTGGGATTCCTGCTGTCAGT |
| LP_DI_D706 | CAAGCAGAAGACGGCATACGAGAT | ACGAATTC | GTGACTGGAGTTCAGACGTGTGCTCTTCCGATC | T | TCAC | AGTGGGATTCCTGCTGTCAGT |
| LP_DI_D707 | CAAGCAGAAGACGGCATACGAGAT | AGCTTCAG | GTGACTGGAGTTCAGACGTGTGCTCTTCCGATC | T | | AGTGGGATTCCTGCTGTCAGT |
| LP_DI_D708 | CAAGCAGAAGACGGCATACGAGAT | GCGCATTA | GTGACTGGAGTTCAGACGTGTGCTCTTCCGATC | T | C | AGTGGGATTCCTGCTGTCAGT |
| LP_DI_D709 | CAAGCAGAAGACGGCATACGAGAT | CATAGCCG | GTGACTGGAGTTCAGACGTGTGCTCTTCCGATC | T | CT | AGTGGGATTCCTGCTGTCAGT |
| LP_DI_D710 | CAAGCAGAAGACGGCATACGAGAT | TTCGCGGA | GTGACTGGAGTTCAGACGTGTGCTCTTCCGATC | T | GCC | AGTGGGATTCCTGCTGTCAGT |
| LP_DI_D711 | CAAGCAGAAGACGGCATACGAGAT | GCGCGAGA | GTGACTGGAGTTCAGACGTGTGCTCTTCCGATC | T | TCAC | AGTGGGATTCCTGCTGTCAGT |
| LP_DI_D712 | CAAGCAGAAGACGGCATACGAGAT | CTATCGCT | GTGACTGGAGTTCAGACGTGTGCTCTTCCGATC | T | GTCCC | AGTGGGATTCCTGCTGTCAGT |

The following table reports the final primers sequences:

**TABLE 4**

| **SEQ ID NO list_first_primer_[Illumina_prot1_DRS_WGA]** | | |
|---|---|---|
| **SEQ ID NO** | **Primer name** | **Complete primer sequence** |
| SEQID N0:195 | LP_DI_D501 | 5'-AATGATACGGCGACCACCGAGATCTACACTATAGCCTACACTCTTTCCCTACACGACGCTCTTCCGATCTAGTGGGATTCCTGCTGTCAGT-3' |
| SEQID NO:196 | LP_DI_D502 | 5'-AATGATACGGCGACCACCGAGATCTAGACATAGAGGGAGACTCTTTCCCTACACGACGCTCTTCCGATCTTAGTGGGATTCCTGCTGTCAGT-3' |
| SEQID NO:197 | LP_DI_D503 | 5'-AATGATACGGCGACCACCGAGATCTAGACCCTATCCTAGACTCTTTCCCTACACGACGCTCTTCCGATCTCTAGTGGGATTCCTGCTGTCAGT-3' |
| SEQID NO:198 | LP_DI_D504 | 5'-AATGATACGGCGACCACCGAGATCTAGACGGCTCTGAACACTCTTTCCCTACACGACGCTCTTCCGATCTGCCAGTGGGATTCCTGCTGTCAGT-3' |
| SEQID NO:199 | LP_DI_D505 | 5'-AATGATACGGCGACCACCGAGATCTACACAGGCGAAGACACTCTTTCCCTACACGACGCTTTCCGATCTGTCCCAGTGGGATTCCTGCTGTCAGT-3' |
| SEQID NO:200 | LP_DI_D506 | 5'-AATGATACGGCGACCACCGAGATCTACACTAATCTTAACACTCTTTCCCTACACGACGCTCTTCCGATCTTCACAGTGGGATTCCTGCTGTCAGT-3' |
| SEQID NO:201 | LP_DI_D507 | 5'-AATGATACGGCGACCACCGAGATCTAGACCAGGACGTACACTCTTTCCCTACACGACGCTCTTCCGATCTAGEGGGATTCCTGCTGTCAGT-3' |
| SEQID NO:202 | LP_DI_D508 | 5'-AATGATACGGCGACCACCGAGATCTAGACGTACTGACACACTCTTTCCCTAGACGACGCTCTTCCGATCTGAGEGGGATTCCTGCTGTCAGT-3' |

| **SEQ ID NO list**_**second_primer_[Illumina_protl1**_**DRS_WGA]** | | |
|---|---|---|
| | **Primer name** | **Complete primer sequence** |
| SEQ ID NO:203 | LP_DI_D701 | 5'-CAAGCAGAAGACGGCATACGAGATCGAGTAATGTGACTGGAGTTCAGACGTGTGCTCTTCCGATCTAGTGGGATTCCTGCTGTCAGT-3' |
| SEQ ID NO:204 | LP_DI_D702 | 5'-GAAGGAGAAGACGGGATACGAGATTCTCCGGAGTGACTGGAGTTCAGACGTGTGCTCTTCCGATCTTAGTGGGATTCCTGCTGTCAGT-3' |
| SEQ ID NO:205 | LP_DI_D703 | 5'-CAAGCAGAAGACGGCATACGAGATAATGAGCGGTGACTGGAGTTCAGACGTGTGCTCTTCCGATCTCTAGTGGGATTCCTGCTGTG4GT-3' |
| SEQ ID NO:206 | LP_DI_D704 | 5'-CAAGCAGAAGACGGCATACGAGATGGAATCTCGTGACTGGAGTTCAGACGTGTGCTCTTCCGATCTGCCAGTGGGATTCCTGCTGTCAGT-3' |
| SEQ ID NO:207 | LP_DI_D705 | 5'-CAAGCAGAAGACGGCATACGAGATTTCTGAATGTGACTGGAGTTG4GACGTGTGCTCTTCCGATCTGTCCG4GTGGGATTCCTGCTGTCAGT-3' |
| SEQ ID NO:208 | LP_DI_D706 | 5'-CAAGCAGAAGACGGCATACGAGATACGAATTCGTGACTGGAGTTCAGACGTGTGCTCTTCCGATCTTCACAGTGGGATTCCTGCTGTGAGT-3' |
| SEQ ID NO:209 | LP_DI_D707 | 5'-CAAGCAGAAGACGGCATACGAGATAGCTTCAGGTGACTGGAGTTCAGACGTGTGCTCTTCCGATCTAGTGGGATTCCTGCAGTCAGT-3' |
| SEQ ID NO:210 | LP_DI_D708 | 5'-CAAGCAGAAGACGGCATACGAGATGCGCATTAGTGACTGGAGTTCAGACGTGTGCTCTTCCGATCTGAGTGGGATTCCTGCTGTCAGT-3' |
| SEQ ID NO:211 | LP_DI_D709 | 5'-CAAGCAGAAGACGGCATACGAGATCATAGCCGGTGACTGGAGTTCAGACGTGTGCTCTTCCGATCTCTAGTGGGATTCCTGCTGTCAGT-3' |
| SEQ ID NO:212 | LP_DI_D710 | 5'-CAAGCAGAAGACGGCATACGAGATTTCGCGGAGTGACTGGAGTTCAGACGTGTGCTCTTCCGATCTGCCAGTGGGATTCCTGCTGTGAGT-3' |
| SEQ ID NO:213 | LP_DI_D711 | 5'-CAAGCAGAAGACGGCATACGAGATGCGCGAGAGTGACTGGAGTTCAGACGTGTGCTCTTCCGATCTTCACAGTGGGATTCCTGCTGTCAGT-3' |
| SEQ ID NO:214 | LP_DI_D712 | 5'-GAAGGAGAAGACGGGATACGAGATCTATCGCTGEGACTGGAGTTCAGACGTGrGCTCTTCCGATCTGrCCCAGTGGGATTCCTGCTGTCAGT-3' |

**SEQ ID NO: list_first_primer_[ILLUMINA/MALBAC] protocol1**

The following table illustrate the structure of the primers MALBAC-WGA compatible for Illumina platform
(sequences in 5' → 3' direction, 5' and 3' omitted):

**TABLE 5**

| primer name | PS/primerindex×2 | 5 | primer read1 | tailing | space | MALBAC |
|---|---|---|---|---|---|---|
| LP_M_0501 | AATGATACGGCGACCACCGAGATCTACAC | TATAGCCT | ACACTCTTTCCCTACACGACGCTCTTCCGATCT | | | GTGAGTGATGGTTGAGGTAGTGTGGAG |
| LP_M_0502 | AATGATACGGCGACCACCGAGATCTACAC | ATAGAGGC | ACACTCTTTCCCTACACGACGCTCTTCCGATCT | | T | GTGAGTGATGGTTGAGGTAGTGTGGAG |
| LP_M_0503 | AATGATACGGCGACCACCGAGATCTACAC | CCTATCCT | ACACTCTTTCCCTACACGACGCTCTTCCGATCT | | CT | GTGAGTGATGGTTGAGGTAGTGTGGAG |
| LP_M_0504 | AATGATACGGCGACCACCGAGATCTACAC | GGCTCTGA | ACACTCTTTCCCTACACGACGCTCTTCCGATCT | | GCC | GTGAGTGATGGTTGAGGTAGTGTGGAG |
| LP_M_0505 | AATGATACGGCGACCACCGAGATCTACAC | AGGCGAAG | ACACTCTTTCCCTACACGACGCTCTTCCGATCT | | GTCCC | GTGAGTGATGGTTGAGGTAGTGTGGAG |
| LP_M_0506 | AATGATACGGCGACCACCGAGATCTACAC | TAATCTTA | ACACTCTTTCCCTACACGACGCTCTTCCGATCT | | TCAC | GTGAGTGATGGTTGAGGTAGTGTGGAG |
| LP_M_0507 | AATGATACGGCGACCACCGAGATCTACAC | CAGGACGT | ACACTCTTTCCCTACACGACGCTCTTCCGATCT | | | GTGAGTGATGGTTGAGGTAGTGTGGAG |
| LP_M_0508 | AATGATACGGCGACCACCGAGATCTACAC | GTACTGAC | ACACTCTTTCCCTACACGACGCTCTTCCGATCT | | C | GTGAGTGATGGTTGAGGTAGTGTGGAG |

| primer name | P7 | i7c | primer read 2 | | | MALBAC |
|---|---|---|---|---|---|---|
| LP_M_0701 | CAAGCAGAAGACGGCATACGAGAT | CGAGTAAT | GTGACTGGAGTTCAGACGTGTGCTCTTCCGATC | T | | GTGAGTGATGGTTGAGGTAGTGTGGAG |
| LP_M_0702 | CAAGCAGAAGACGGCATACGAGAT | TCTCCGGA | GTGACTGGAGTTCAGACGTGTGCTCTTCCGATC | T | T | GTGAGTGATGGTTGAGGTAGTGTGGAG |
| LP_M_0703 | CAAGCAGAAGACGGCATACGAGAT | AATGAGCG | GTGACTGGAGTTCAGACGTGTGCTCTTCCGATC | T | CT | GTGAGTGATGGTTGAGGTAGTGTGGAG |
| LP_M_0704 | CAAGCAGAAGACGGCATACGAGAT | GGAATCTC | GTGACTGGAGTTCAGACGTGTGCTCTTCCGATC | T | GCC | GTGAGTGATGGTTGAGGTAGTGTGGAG |
| LP_M_0705 | CAAGCAGAAGACGGCATACGAGAT | TTCTGAAT | GTGACTGGAGTTCAGACGTGTGCTCTTCCGATC | T | GTCCC | GTGAGTGATGGTTGAGGTAGTGTGGAG |
| LP_M_0706 | CAAGCAGAAGACGGCATACGAGAT | ACGAATTC | GTGACTGGAGTTCAGACGTGTGCTCTTCCGATC | T | TCAC | GTGAGTGATGGTTGAGGTAGTGTGGAG |
| LP_M_0707 | CAAGCAGAAGACGGCATACGAGAT | AGCTTCAG | GTGACTGGAGTTCAGACGTGTGCTCTTCCGATC | T | | GTGAGTGATGGTTGAGGTAGTGTGGAG |
| LP_M_0708 | CAAGCAGAAGACGGCATACGAGAT | GCGCATTA | GTGACTGGAGTTCAGACGTGTGCTCTTCCGATC | T | C | GTGAGTGATGGTTGAGGTAGTGTGGAG |
| LP_M_0709 | CAAGCAGAAGACGGCATACGAGAT | CATAGCCG | GTGACTGGAGTTCAGACGTGTGCTCTTCCGATC | T | CT | GTGAGTGATGGTTGAGGTAGTGTGGAG |
| LP_M_0710 | CAAGCAGAAGACGGCATACGAGAT | | GTGACTGGAGTTCAGACGTGTGCTCTTCCGATC | T | GCC | GTGAGTGATGGTTGAGGTAGTGTGGAG |
| LP_M_0711 | CAAGCAGAAGACGGCATACGAGAT | GCGCGAGA | GTGACTGGAGTTCAGACGTGTGCTCTTCCGATC | T | TCAC | GTGAGTGATGGTTGAGGTAGTGTGGAG |
| LP_M_0712 | CAAGCAGAAGACGGCATACGAGAT | CTATCGCT | GTGACTGGAGTTCAGACGTGTGCTCTTCCGATC | T | GTCCC | GTGAGTGATGGTTGAGGTAGTGTGGAG |

The following table reports the final primers sequences:

**TABLE 6**

| **SEQ ID NO list_first_primer_[Illumina_prot1/MALBAC]** | | |
|---|---|---|
| **SEQ ID NO:** | **Primer Name** | **Complete primer sequence** |
| SEQID NO:215 | LP_MI_D501 | 5'-AATGATACGGCGACCACCGAGATCTACACTATAGCCTACACTCTTTCCCTACACGACGCTCTTCCGATCTGTGAGTGATGGTTGAGGTAGTGTGGAG-3' |
| SEQID NO:216 | LP_MI_D502 | 5'-AATGATACGGCGACCACCGAGATCTACACATAGAGGCACACTCTTTCCCTACACGCTCTTCCGATCTTGTGAGTGATGGTTGAGGTAGTGTGGAG-3' |
| SEQID NO:217 | LP_MI_D503 | 5'-AATGATACGGCGACCACCGAGATCTACACCCTATCCTACACTCTTTCCCTACACGACGCTCTTCCGATCTCTGTTGAGTGATGGTTGAGGTAGTGTGGAG-3' |
| SEQID NO:218 | LP_MI_D504 | 5'-AATGATACGGCGACCACCGAGATCTACACGGCTCTGAACACTCTTTCCCTACACGACGCCTCTTCCGATCTGCCGTGAGTGATGGTTGAGGTAGTGTGGAG-3' |
| SEQID NO:219 | LP_MI_D505 | 5'-AATGATACGGCGACCACCGAGATCTACACAGGCGAAGACACTCTTTCCCTACACGACGCTCTTCCGATCTGTCCCGTGAGTGATGGTTGAGGTAGTGGAG-3' |
| SEQID NO:220 | LP_MI_D506 | 5'-AATGATACGGCGACCACCGAGATCTACACTAATCTTAACACTCTTTCCCTACACGACGCTCTTCCGATCTTCACGTGAGTGATGGTTGAGGTAGTGTGGAG-3' |
| SEQID NO:221 | LP_MI_D507 | 5'-AATGATACGGCGACCACCGAGATCTACACCAGGACGTACACTCTTTCCCTACACGACGCTCTTCCGATCTGTGATGGTTGAGGTAGTGTGGAG-3' |
| SEQID NO:222 | LP_MI_D508 | 5'-AATGATACGGCGACCACCGAGATCTACACGTACTGACACTCTTTCCCTACACGACGCTCTTCCGATCTCGTGAGTGATGGTTGAGGTAGTGTGGAG-3' |

| **SEQ ID NO list_second_primer_[Illumina_prot1/MALBAC]** | | |
|---|---|---|
| SEQ ID NO:223 | LP_MI_D701 | 5'-CAAGCAGAAGACGGCATACGAGATCGAGTAATGTGACTGGAGTTCAGACGTGTGCTCTTCCGATCTGTGAGTGATGGTTGAGGTAGTGTGGAG-3' |
| SEQ ID NO:224 | LP_MI_D702 | 5'-CAAGCAGAAGACGGCATACGAGATTCTCCGGAGTGACTGGAGTTCAGACGTGTGCTCTTCCGATCTTGTGAGTGATGGTTGAGGTAGTGTGGAG-3' |
| SEQ ID NO:225 | LP_MI_D703 | 5'-CAAGCAGAAGACGGCATACGAGATAATGAGCGGTGACTGGAGTTCAGACGTGTGCTCTTCCGATCTCTGTGAGTGATGGTTGAGGTAGTGTGGAG-3' |
| SEQ ID NO:226 | LP_MI_D704 | 5'-CAAGCAGAAGACGGCATACGAGATGGAATCTCGTGACTGGAGTTCAGACGTGTGCTCTTCCGATCTGCCGTGAGTGATGGTTGAGGTAGTGTGGAG-3' |
| SEQ ID NO:227 | LP_MI_D705 | 5'-CAAGCAGAAGACGGCATACGAGATTTCTGAATGTGACTGGAGTTCAGACGTGTGCTCTTCCGATCTGTCCCGTGAGTGATGGTTGAGGTAGTGTGGAG-3' |
| SEQ ID NO:228 | LP_MI_D706 | 5'-CAAGCAGAAGACGGCATACGAGATACGAATTCGTGACTGGAGTTCAGACGTGTGCTCTTCCGATCTTCACGTGAGTGATGGTTGAGGTAGTGTGGAG-3' |
| SEQ ID NO:229 | LP_MI_D707 | 5'-CAAGCAGAAGACGGCATACGAGATAGCTTCAGGTGACTGGAGTTCAGACGTGTGCTCTTCCGATCTGTGAGTGATGGTTGAGGTAGTGTGGAG-3' |
| SEQ ID NO:230 | LP_MI_D708 | 5'-CAAGCAGAAGACGGCTACGAGATGCGCATTAGTGACTGGAGTTCAGACGTGTGCTCTTCCGATCTCGTGAGTGATGGTTGAGGTAGTGTGGAG-3' |
| SEQ ID NO:231 | LP_MI_D709 | 5'-CAAGCAGAAGACGGCATACGAGATCATAGCCGGTGACTGGAGTTCAGACGTGTGCTCTTCCGATCTCTGTGAGTGATGGTTGAGGTAGTGTGGAG-3' |
| SEQ ID NO:232 | LP_MI_D710 | 5'-CAAGCAGAAGACGGCATACGAGATTTCGCGGAGTGACTGGAGTTCAGACGTGTGCTCTTCCGATCTGCCGTGAGTGATGGTTGAGGTAGTGTGGAG-3' |
| SEQ ID NO:233 | LP_MI_D711 | 5'-CAAGCAGAAGACGGCATACGAGATGCGCGAGAGAGTGACTGGAGTTCAGACGTGTGCTCTTCCGATCTTCACGTGAGTGATGGTTGAGGTAGTGTGGAG-3' |
| SEQ ID NO:234 | LP_MI_D712 | 5'-CAAGCAGAAGACGGCATACGAGATCTATCGCTGTGACTGGAGTTCAGACGTGTGCTCTTCCGATCTGTCCCGTGAGTGATGGTTGAGGTAGTGTGGAG-3' |

### Limitations of protocol 1

The libraries resulting from Illumina protocol 1 are double stranded pWGA lib with all possible P5/P7 adapter combination couples.

Since within the flow cell the hybridization occurred as well by fragments with homogenous sequencing adapters (P5/P5rc, P7rc/P7), the cluster density and/or quality of clusters could result slightly lower compared to the case Illumina protocol 2.

### Protocol 2

A second protocol is provided by way of example. This protocol may be of advantage to increase the quality of clusters in the Illumina flow-cells, by selecting from the pWGAlib only fragments which encompass both sequencing adapters (P5/P7), discarding fragments with homogenous sequencing adapters (P5/P5rc, P7rc/P7).

### Workflow Description of Protocol 2 (Illumina/DRS_WGA) as schematically illustrated in Figure 4

All WGA-amplified DNA products are composed by molecules different in length, and with a specific tag: the LIB sequence in 5' end and the complementary LIB sequence on 3' end of each individual ssDNA molecule (indicated in blue in the figure).

According to this protocol both reverse complement LIB sequence are the targets for the NGS Re-Amp (re-amplification) primers.

Two type of primers have been designed: LPb_DI_D50X (range between SEQ ID NO:235 to SEQ ID NO:242 primer) and biotinylated primer LPb_DI_D70X (range between SEQ ID NO:243 to SEQ ID NO:254 primer), respectively in green-yellow-blue and in red-pink-blue in the figure.

As expected, both type of primers may bind the LIB sequence and the complementary LIB sequence, and as matter of fact three types of amplicons arise from the NGS Re-Amp process, as indicated in the figure.

This protocol is provided by LPb_DI_D70X (indicated in the figure as P7rc adapter) that get a biotin tag on 5' end. This specific tag is used to select, by streptavidin beads, the only one fragment without biotin tag:
5'-P5-i5-LIB-insert-LIBcomplementary-i7-P7-3'
as illustrated in the figure.

To obtain ssDNA of the wanted formation (omitting for the sake of simplicity the read primers sections, wanted ssDNA is: 5'-P5-i5-nnnnn-i7-P7-3'), primers shall be like:
(1PR) 5'-P5-i5-LIB-3' and
(2PR) Biotyn-5'-P7rc-i7rc-LIB-3' (Biotin will be omitted in what follows for the sake of simplicity of description, but it is apparent that it will be present in all and only the 5' ends of fragments starting with P7rc) .

Through re-amplification it is obtained:
start: (the WGA ssDNA fragments are all formed as: 5'-LIB-nnn-LIBrc-3')
- extension cycle n=1):

| | |
|---|---|
| 1.5 | 5'-P5-i5-LIB-nnn-LIBrc-3', |

1.7 5'-P7rc-i7rc-LIB-nnn-LIBrc-3'
2^n frags [0% sequencable]

- cycle n=2):

| | |
|---|---|
| 2.5.5 | 5'-P5-i5-LIB-nnn-LIBrc-i5rc-P5rc-3' |
| 2.5.7 | 5'-P7rc-i7rc-LIB-nnn-LIBrc-i5rc-P5rc-3' |
| 2.7.5 | 5'-P5-i5-LIB-nnn-LIBrc-i7-P7-3' |
| 2.7.7 | 5'-P7rc-i7rc-LIB-nnn-LIBrc-i7-P7-3' |

2^n=4 frags[25% sequenceable frags]
- cycle n=3):

| | | |
|---|---|---|
| 2.5.5.5 | 5'-P5-i5-LIB-nnn-LIBrc-i5rc-P5rc-3' | = 2.5.5 |
| 2.5.5.7 | 5'-P7rc-i7rc-LIB-nnn-LIBrc-i5rc-P5rc-3' | = 2.5.7 |
| 2.5.7.5 | 5'-P5-i5-LIB-nnn-LIBrc-i7-P7-3' | = 2.7.5 |
| 2.5.7.7 | 5'-P7rc-i7rc-LIB-nnn-LIBrc-i7-P7-3' | = 2.7.7 |
| 2.7.5.5 | 5'-P5-i5-LIB-nnn-LIBrc-i5rc-PSrc-3' | = 2.5.5 |
| 2.7.5.7 | 5'-P7rc-i7rc-LIB-nnn-LIBrc-i5rc-PSrc-3' | = 2.5.7 |
| 2.7.7.5 | 5'-P5-i5-LIB-nnn-LIBrc-i7-P7-3' | = 2.7.5 |
| 2.7.7.7 | 5'-P7rc-i7rc-LIB-nnn-LIBrc-i7-P7-3' | = 2.7.7 |

2^n=8 frags [25% sequenceable frags] 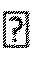 sequenceable frags = 2^n/4 = 2^n/2^2 = 2^(n-2)
Cycle m) ... 2^(m-2) sequenceable

In the end the following four types of fragments are formed after exponential amplification.
2.5.5 5'-P5-i5-LIB-nnn-LIBrc-i5rc-PSrc-3' (→ will be washed out at first liquid removal, while holding all biotinylated fragments on the paramagnetic beads or -if not washed out- will engage only one binding site in the flow-cell but doesn't generate a sequencing cluster as no bridge amplification occurs).
2.5.7 Biotyn-5'-P7rc-i7rc-LIB-nnn-LIBrc-i5rc-P5rc-3' (→ will be removed by streptavidin coated beads)
2.7.5 5'-P5-i5-LIB-nnn-LIBrc-i7-P7-3' (→ sequenceable)
2.7.7 Biotyn-5'-P7rc-i7rc-LIB-nnn-LIBrc-i7-P7-3' (-> will be removed by streptavidin coated beads).

### Example 4:

### 1. Deterministic-Restriction Site Whole genome amplification (DRS-WGA)

Single cell DNA was amplified using the *Ampli1*^{™} WGA Kit (Silicon Biosystems) according to the manufacturer's instructions.

### 2. Re-amplification of the WGA products

Five µL of WGA-amplified DNA are diluted by addition of 5 µL of Nuclease-Free Water and purified using Agencourt AMPure XP system (Beckman Coulter) in order to remove unbound oligos and excess nucleotides, salts and enzymes.

The beads-based DNA purification was performed according to the following protocol: 18 µL of beads (1.8X sample volume) were added to each sample. Beads and reaction products were mixed by briefly vortexing and then spin-down to collect the droplets. Mixed reactions were then incubated off-magnet for 15 minutes at room temperature, after which they were then transferred to a DynaMag-96 Side magnet (Life Technologies) and left to stand for 5 min. Supernatant were discarded and beads washed with 150 µL of freshly made 80% EtOH. After a second round of EtOH washing, beads were allowed to dry on the magnet for 5-10 min. Dried beads were then resuspended off-magnet in 15 µL of Low TE buffer and incubated for 10 min, followed by 5 min incubation on-magnet. Twelve microliters of the eluate were transferred to another tube and subsequently quantified by dsDNA HS Assay on the Qubit^{®} 2.0 Fluorometer in order to prepare aliquots of 10 µL containing 25 ng of WGA-purified DNA.

Barcoded re-amplification was performed in a volume of 50 µl using Ampli1^{™} PCR Kit (Silicon Biosystems). Each PCR reaction was composed as following:
5 µl Ampli1^{™} PCR Reaction Buffer (10X), 1µL of 25 µM of one primer of SEQ ID NO:235 to SEQ ID NO:242
[3] 5'AATGATACGGCGACCACCGAGATCTACAC[i5]GCTCTCCGTAGTGGGATTCCTGCTGTCAGTTAA3')
   1µL of 25 µM of one primer of SEQ ID NO:243 to SEQ ID NO:254
[4] (5'/Biosg/CAAGCAGAAGACGGCATACGAGAT[i7]GCTCACCGAAGTGGGATTCCTGCTGTCAGTTAA3')
   1.75 µl Ampli1^{™} PCR dNTPs (10 mM), 1.25 µl BSA, 0.5 Ampli1^{™} PCR Tag Polymerase and 25 ng of the WGA-purified DNA and 37.5 µl of Ampli1^{™} Water.

Applied Biosystems^{®} 2720 Thermal Cycler was set as follows: 95°C for 4 min, 1 cycle of 95°C for 30 sec, 60°C for 30 sec, 72°C for 2 min, 10 cycles of 95°C for 30 sec, 60°C for 30 sec, 72°C for 2 min (extended by 20 sec/cycle) and final extension at 72° C for 7 min.

### 3) Size selection

Barcoded re-amplified WGA products, correspondent to a fragment library with provided Illumina adapters, were qualified by Agilent DNA 7500 Kit on the 2100 Bioanalyzer^{®} (Agilent) and quantified using Qubit^{®} dsDNA HS Assay Kit in order to obtain a final pool.

The equimolar pool was created by combining the same amount of individual libraries with different LPb_DI dual index adapter, producing the final pool with the concentration of 35 ng/µL in a final volume of 50 µL. The concentration of the pool was confirmed by the Qubit^{®} method.

A fragments section range between 200bp to 1 Kb has been selected by double purification utilizing SPRI beads system (Beckman Coulter) with ratio R:0.47X and L:0.85X respectively. In order to remove large DNA fragment we added 82 µL of diluted SPRI (42 µL SPRI bead + 42 µL PCR grade water) and 34.2 µL of undiluted SPRI bead to the supernatant to remove small DNA fragments.

Final library was eluted in 50 µl of Low TE buffer and evaluated using a 2100 Bioanalyzer High Sensitivity Chip (Agilent Technologies).

### 4) Heterogeneous P5/P7 adapter single strand library selection

A fragment selection has been perform using Dynabeads^{®} MyOne^{™} Streptavidin C1 system, in order to dissociate only non-biotinylated DNA containing P5/P7 adapter and this could be obtained using heat or NaOH respectively. Two methods are described below.

Twenty µL of Dynabeads^{®} MyOne^{™}Streptavidin C1 in a 1,5 ml tube was washed twice with the B&W solution 1X (10 mM Tris-HCl (pH 7.5);1 mM EDTA; 2 M NaCl).

Fifty µL of fractionated pool library was added to Dynabeads^{®} MyOne^{™}Streptavidin C1 bead and incubated for 15 min, pipetting up down every 5 min to mix thoroughly. Wash twice the DNA coated Dynabeads^{®} in 50 µL 1 × SSC (0.15 M NaCl, 0.015 M sodium citrate) and resuspended the beads with fresh 50 µL of 1 × SSC.

After incubation at 95 °C for 5 minutes, the tube was allocated in the magnetic plate for 1 min and the 50 µL of supernatant transferred in a new tube and incubated on ice for 5 min.

In this point the supernatant contains non-biotinylated DNA strands library with P5/P7 adapter.

To ensure that the washing was more stringent, the streptavidin selection procedure was repeat for a second time.

Instead use heat, the washed DNA coated Dynabeads^{®} could be done by resuspending with 20 µl of freshly prepared 0.15 M NaOH.

After incubation at room temperature for 10 min, the tube was allocated in magnet stand for 1-2 minutes and transfer the supernatant to a new tube.

The supernatant contains your non-biotinylated DNA strand. The single strand library was neutralized by adding 2.2 µL 10 × TE, pH 7.5 and 1.3 µL 1.25 M acetic acid.

The final library concentration as quantified by Qubit^{®} ssDNA Assay Kit was 5ng/ µL corresponding to 25 µM.

### 5) MiSeq Sequencing

4 nM of the final pool was denatured 5 minutes with NaOH (NaOH final concentration equal to 0.1N). Denatured sample was then diluted with HT1 to obtain a 20 pM denatured library in 1 mM NaOH. 570 µL of 20 pM denatured library and 30 µl of 20 pM denatured PhiX control were loaded on a MiSeq (Illumina).

Single end read of 150 base were generated using the v3 chemistry of the Illumina MiSeq exchanging the standard Read 1 primer and standard primer index 1 with respectively 600 µL of SEQ ID NO:255 primer (Custom Read 1 primer) and 600 µL SEQ ID NO:256 or SEQ ID NO:258 primer (Custom primer index 1a (i7) and 1b (i7))
**SEQ ID NO list_first_primer_[ILLUMINA_DRS_WGA] protoco2**

The following table reports the final primers sequences of the Illumina protocol 2:

**TABLE 7**

| **SEQID** | **Name** | **Primer sequence** |
|---|---|---|
| **SEQ ID NO list_first_primer_[Illumina_DRS_WGA_prot2]** | | |
| SEQ ID NO:235 | LPb_DI_D501 | 5'-AATGATACGGCGACCACCGAGATCTACACTATAGCCTGCTCTCCGTAGTGGGATTCCTGCTGTCAGTTAA-3' |
| SEQ ID NO:236 | LPb_DI_D502 | 5'-AATGATACGGCGACCACCGAGATCTACACATAGAGGCGCTCTCCGTAGTGGGATTCCTGCTGTCAGTTAA-3' |
| SEQ ID NO:237 | LPb_DI_D503 | 5'-AATGATACGGCGACCACCGAGATCTACACCCTATCCTGCTCTCCGTAGTGGGATTCCTGCTGTCAGTTAA-3' |
| SEQ ID NO:238 | LPb_DI_D504 | 5'-AATGATACGGCGACCACCGAGATCTACACGGCTCTGAGCTCTCCGTAGTGGGATTCCTGCTGTCAGTTAA-3' |
| SEQ ID NO:239 | LPb_DI_D505 | 5'-AATGATACGGCGACCACCGAGATCTACACAGGCGAAGGCTCTCCGTAGTGGGATTCCTGCTGTCAGTTAA-3' |
| SEQ ID NO:240 | LPb_DI_D506 | 5'-AATGATACGGCGACCACCGAGATCTACACTAATCTTAGCTCTCCGTAGTGGGATTCCTGCTGTCAGTTAA-3' |
| SEQ ID NO:241 | LPb_DI_D507 | 5'-AATGATACGGCGACCACCGAGATCTACACCAGGACGTGCTCTCCGTAGTGGGATTCCTGCTGTCAGTTAA-3' |
| SEQ ID NO:242 | LPb_DI_D508 | 5'-AATGATACGGCGACCACCGAGATCTACACGTACTGACGCTCTCCGTAGTGGGATTCCTGCTGTCAGTTAA-3' |

| **SEQ ID NO list_second_primer_[Illumina_DRS_WGA_prot2]** | | |
|---|---|---|
| SEQ ID NO:243 | LPb_DI_D701 | /5Biosg/CAAGCAGAAGACGGCATACGAGATCGAGTAATGCTCACCGAAGTGGGATTCCTGCTGTCAGTTAA-3' |
| SEQ ID NO:244 | LPb_DI_D702 | /5Biosg/CAAGCAGAAGACGGCATACGAGATTCTCCGGAGCTCACCGAAGTGGGATTCCTGCTGTCAGTTAA-3' |
| SEQ ID NO:245 | LPb_DI_D703 | /5Biosg/CAAGCAGAAGACGGCATACGAGATAATGAGCGGCTCACCGAAGTGGGATTCCTGCTGTCAGTTAA-3' |
| SEQ ID NO:246 | LPb_DI_D704 | /5Biosg/CAAGCAGAAGACGGCATACGAGATGGAATCTCGCTCACCGAAGTGGGATTCCTGCTGTCAGTTAA-3' |
| SEQ ID NO:247 | LPb_DI_D705 | /5Biosg/CAAGCAGAAGACGGCATACGAGATTTCTGAATGCTCACCGAAGTGGGATTCCTGCTGTCAGTTAA-3' |
| SEQID248 | LPb_DI_D706 | /5Biosg/CAAGCAGAAGACGGCATACGAGATACGAATTCGCTCACCGAAGTGGGATTCCTGCTGTCAGTTAA-3' |
| SEQ ID NO:249 | LPb_DI_D707 | /5Biosg/CAAGCAGAAGACGGCATACGAGATAGCTTCAGGCTCACCGAAGTGGGATTCCTGCTGTCAGTTAA-3' |
| SEQ ID NO:250 | LPb_DI_D708 | /5Biosg/CAAGCAGAAGACGGCATACGAGATGCGCATTAGCTCACCGAAGTGGGATTCCTGCTGTCAGTTAA-3' |
| SEQ ID NO:251 | LPb_DI_D709 | /5Biosg/CAAGCAGAAGACGGCATACGAGATCATAGCCGGCTCACCGAAGTGGGATTCCTGCTGTCAGTTAA-3' |
| SEQ ID NO:252 | LPb_DI_D710 | /5Biosg/CAAGCAGAAGACGGCATACGAGATTTCGCGGAGCTCACCGAAGTGGGATTCCTGCTGTCAGTTAA-3' |
| SEQ ID NO:253 | LPb_DI_D711 | /5Biosg/CAAGCAGAAGACGGCATACGAGATGCGCGAGAGCTCACCGAAGTGGGATTCCTGCTGTCAGTTAA-3' |
| SEQ ID NO:254 | LPb_DI_D712 | /5Biosg/CAAGCAGAAGACGGCATACGAGATCTATCGCTGCTCACCGAAGTGGGATTCCTGCTGTCAGTTAA-3' |

| **SEQ ID NO list_SB Custom Sequencing Primer_[Illumina_DRS_WGA_prot2]** | | |
|---|---|---|
| SEQ ID NO:255 | Custom Read 1 primer | 5'-GCTCTCCGTAGTGGGATTCCTGCTGTCAGTTAA-3' |
| SEQ ID NO:256 | Custom primer index 1a (i7) | 5'-TTAACTGACAGCAGGAATCCCACTACGGAGAGC-3' |
| SEQ ID NO:257 | Custom primer read 2 (optional) | 5'-GCTCACCGAAGTGGGATTCCTGCTGTCAGTTAA-3' |
| SEQ ID NO:258 | Custom primer index 1b (i7) | 5'-**TTAACTGACAGCAGGAATCCCACTTCGGTGAGC**-3' |

| | | |
|---|---|---|
| **SEQ ID NO list_first_primer_[ILLUMINA/MALBAC] protocol2** | | |

The following table reports the final primers sequences Illumina compatible in case the starting material comes from a WGA-MALBAC library:

**TABLE 8**

| SEQ ID NO | **Name** | **Primer sequence** |
|---|---|---|
| **SEQ ID NO list_first_primer_[Illumina/MALBAC_prot2]** | | |
| SEQ ID NO:259 | LP_MII_D501 | 5'-AATGATACGGCGACCACCGAGATCTACACTATAGCCTGTGAGTGATGGTTGAGGTAGTGTGGAG-3' |
| SEQ ID NO:260 | LP_MII_D502 | 5'-AATGATACGGCGACCACCGAGATCTACACATAGAGGCGTGAGTGATGGTTGAGGTAGTGTGGAG-3' |
| SEQ ID NO:261 | LP_MII_D503 | 5'-AATGATACGGCGACCACCGAGATCTACACCCTATCCTGTGAGTGATGGTTGAGGTAGTGTGGAG-3' |
| SEQ ID NO:262 | LP_MII_D504 | 5'-AATGATACGGCGACCACCGAGATCTACACGGCTCTGAGTGAGTGATGGTTGAGGTAGTGTGGAG-3' |
| SEQ ID NO:263 | LP_MII_D505 | 5'-AATGATACGGCGACCACCGAGATCTACACAGGCGAAGGTGAGTGATGGTTGAGGTAGTGTGGAG-3' |
| SEQ ID NO:264 | LP_MII_D506 | 5'-AATGATACGGCGACCACCGAGATCTACACTAATCTTAGTGAGTGATGGTTGAGGTAGTGTGGAG-3' |
| SEQ ID NO:265 | LP_MII_D507 | 5'-AATGATACGGCGACCACCGAGATCTACACCAGGACGTGTGAGTGATGGTTGAGGTAGTGTGGAG-3' |
| SEQ ID NO:266 | LP_MII_D508 | 5'-AATGATACGGCGACCACCGAGATCTACACGTACTGACGTGAGTGATGGTTGAGGTAGTGTGGAG-3' |

| **SEQ ID NO list_second_primer_[Illumina/MALBAC_prot2]** | | |
|---|---|---|
| SEQ ID NO:267 | LP_MII_D701 | /5Biosg/CAAGCAGAAGACGGCATACGAGATCGAGTAATGTGAGTGATGGTTGAGGTAGTGTGGAG-3' |
| SEQ ID NO:268 | LP_MII_D702 | /5Biosg/CAAGCAGAAGACGGCATACGAGATTCTCCGGAGTGAGTGATGGTTGAGGTAGTGTGGAG-3' |
| SEQ ID NO:269 | LP_MII_D703 | /5Biosg/CAAGCAGAAGACGGCATACGAGATAATGAGCGGTGAGTGATGGTTGAGGTAGTGTGGAG-3' |
| SEQ ID NO:270 | LP_MII_D704 | /5Biosg/CAAGCAGAAGACGGCATACGAGATGGAATCTCGTGAGTGATGGTTGAGGTAGTGTGGAG-3' |
| SEQ ID NO:271 | LP_MII_D705 | /5Biosg/CAAGCAGAAGACGGCATACGAGATTTCTGAATGTGAGTGATGGTTGAGGTAGTGTGGAG-3' |
| SEQ ID NO:272 | LP_MII_D706 | /5Biosg/CAAGCAGAAGACGGCATACGAGATACGAATTCGTGAGTGATGGTTGAGGTAGTGTGGAG-3' |
| SEQ ID NO:273 | LP_MII_D707 | /5Biosg/CAAGCAGAAGACGGCATACGAGATAGCTTCAGGTGAGTGATGGTTGAGGTAGTGTGGAG-3' |
| SEQ ID NO:274 | LP_MII_D708 | /5Biosg/CAAGCAGAAGACGGCATACGAGATGCGCATTAGTGAGTGATGGTTGAGGTAGTGTGGAG-3' |
| SEQ ID NO:275 | LP_MII_D709 | /5Biosg/CAAGCAGAAGACGGCATACGAGATCATAGCCGGTGAGTGATGGTTGAGGTAGTGTGGAG-3' |
| SEQ ID NO:276 | LP_MII_D710 | /5Biosg/CAAGCAGAAGACGGCATACGAGATTTCGCGGAGTGAGTGATGGTTGAGGTAGTGTGGAG-3' |
| SEQ ID NO:277 | LP_MII_D711 | /5Biosg/CAAGCAGAAGACGGCATACGAGATGCGCGAGAGTGAGTGATGGTTGAGGTAGTGTGGAG-3' |
| SEQ ID NO:278 | LP_MII_D712 | /5Biosg/CAAGCAGAAGACGGCATACGAGATCTATCGCTGTGAGTGATGGTTGAGGTAGTGTGGAG-3' |

| **SEQ ID NO list_SB Custom Sequencing Primer_[Illumina/MALBAC_prot2]** | | |
|---|---|---|
| SEQ ID NO:279 | Custom Read 1M primer | 5'-GTGAGTGATGGTTGAGGTAGTGTGGAG-3' |
| SEQ ID NO:280 | Custom primer index 1M (i7) | 5'-CTCCACACTACCTCAACCATCACTCAC-3' |
| SEQ ID NO:281 | Custom primer read 2M (optional) | 5'-GCTCACCGAAGTGGGATTCCTGCTGTCAGTTAA-3' |

Both LIB reverse complementary are the targets for the NGS Re-Amp (re-amplification) primers as shown in the figure 4. Furthermore, a custom read1 sequencing primer (SEQ ID NO:255) has been designed, in order to increase the library complexity, because the reads will not start with the same nucleotide that could affect the sequencing performance or avoid use a high concentration spike-in to ensure more diverse set of clusters for matrix, phasing, and prephasing calculations. The custom read1 sequencing primer (SEQ ID NO:255) contains the LIB sequence and it is complementary to the LIB reverse complement sequence, as illustrated in Figure 4.

Moreover, the NGS Re-Amp (re- amplification) products don't have the canonical sequence used by Illumina systems to read the index 1, for this reason it is needed to use custom sequencing primer index 1 (i7) (SEQ ID NO:256 or SEQ ID NO:258) to allow the correct reading of index i7. Noteworthy is that the custom sequencing primer index 1 contains the reverse complementary LIB sequence.

All the examples described above which include procedures PGM/Proton and Illumina protocol 1/2 workflow, could be performed using primer MALBAC compatible listed in the tables above (SEQ ID NO:98 to SEQ ID NO:194 and SEQ ID NO:215 to SEQ ID NO:234 and SEQ ID NO:259 to SEQ ID NO:281) .

### Data analysis

Sequenced reads were aligned to the hg19 human reference genome using the BWA MEM algorithm (Li H. and Durbin R., 2010). PCR duplicates, secondary/supplementary/not-passing-QC alignments and multimapper reads were filtered out using Picard MarkDuplicates (http://broadinstitute.github.io/picard/) and samtools (Li H. et al, 2009). Coverage analyses were performed using BEDTools (Quinlan A. et al, 2010).

Control-FREEC (Boeva V. et al., 2011) algorithm was used to obtain copy-number calls without a control sample. Read counts were corrected by GC content and mappability (uniqMatch option) and window size were determined by software using coefficientOfVariation = 0.06. Ploidy was set to 2 and contamination adjustment was not used.

Plots for CNV profiles were obtained using a custom python script as shown in Figures from 6 to 9.

Although the present invention has been described with reference to the method for Ampli1 WGA only, the technique described, as it appears obvious for one skilled in the art, clearly applies *mutatis mutandis* also to any other kind of WGA (e.g. MALBAC) which comprise a library with self-complementary 5' and 3' regions.

## Claims

1. A method for low-pass whole genome sequencing comprising the steps of:
- providing a plurality of barcoded, massively-parallel sequencing libraries obtained by a method of generating a massively parallel sequencing library comprising the steps of:
- providing a primary WGA DNA library (pWGAlib) including fragments consisting of a known 5' sequence section (5SS), a middle sequence section (MSS), and a known 3' sequence section (3SS) reverse complementary to the known 5' sequence section, the known 5' sequence section (5SS) consisting of a DRS-WGA library universal sequence adaptor or a MALBAC library universal sequence adaptor, and the middle sequence section (MSS) consisting of at least an insert section (IS), corresponding to a DNA sequence of the original unamplified DNA prior to WGA, and optionally a flanking 5' intermediate section (F5) and/or a flanking 3' intermediate section (F3);
- re-amplifying the primary WGA DNA library using at least one first primer (1PR) and at least one second primer (2PR); wherein
the at least one first primer (1PR) comprises a first primer 5' section (1PR5S) and a first primer 3' section (1PR3S), the first primer 5' section (1PR5S) comprising at least one first sequencing adaptor (1PR5SA) and at least one first sequencing barcode (1PR5BC) in 3' position of the at least one first sequencing adaptor (1PR5SA) and in 5' position of the first primer 3' section (1PR3S), and the first primer 3' section (1PR3S) hybridizing to either the known 5' sequence section (5SS) or the known 3' sequence section (3SS);
the at least one second primer (2PR) comprises a second primer 5' section (2PR5S) and a second primer 3' section (2PR3S), the second primer 5' section (2PR5S) comprising at least one second sequencing adaptor (2PR5SA) different from the at least one first sequencing adaptor (1PR5SA), and the second primer 3' section (2PR3S) hybridizing to either the known 5' sequence section (5SS) or the known 3' sequence section (3SS)
- pooling samples obtained using different sequencing barcodes (BC); and
- sequencing the pooled library.

2. The method according to claim 1, wherein the second primer (2PR) further comprises at least one second sequencing barcode (2PR5BC), in 3' position of the at least one second sequencing adaptor (2PR5SA) and in 5' position of the second primer 3' section (2PR3S).

3. The method according to claim 1 or 2, wherein the DRS-WGA library universal sequence adaptor is SEQ ID NO:282 and the MALBAC library universal sequence adaptor is SEQ ID NO:283 (MALBAC).

4. The method for low-pass whole genome sequencing according to any of claims 1 to 3, wherein the step of pooling samples using different sequencing barcodes (BC) further comprises the steps of:
- quantitating the DNA in each of the barcoded, massively-parallel sequencing libraries;
- normalizing the amount of barcoded, massively-parallel sequencing libraries.

5. The method for low-pass whole genome sequencing according to claim 4, wherein the step of pooling samples using different sequencing barcodes (BC) further comprises the step of selecting DNA fragments having at least one selected range of base pairs, wherein the range of base pairs is centered on 650 bp, or 400 bp, or 200 bp, or 150 bp, or 100 bp, or 50 bp.

6. The method for low-pass whole genome sequencing according to claim 5, further comprising the step of selecting DNA fragments comprising the first sequencing adaptor and the second sequencing adaptors.

7. The method for low-pass whole genome sequencing according to claim 6, wherein the step of selecting DNA fragments comprising the first sequencing adaptor and the second sequencing adaptors is carried out by contacting the massively parallel sequencing library to paramagnetic beads functionalized with a streptavidin coating, and
wherein one of the at least one first primer (1PR) and the at least one second primer (2PR) are biotinylated at the 5' end, and selected fragments are obtained eluting from the beads non-biotinylated ssDNA fragments.

8. The method for low-pass whole genome sequencing according to claim 7, wherein the at least one second primer is biotinylated at 5' end.

9. The method for low-pass whole genome sequencing according to claim 7 or 8, further comprising the further steps of:
- incubating the re-amplified WGA dsDNA library with the functionalized paramagnetic beads under designed conditions thus causing covalent binding between biotin and streptavidin allocated in the functionalized paramagnetic beads;
- washing out unbound non-biotinylated dsDNA fragments;
- eluting from the functionalized paramagnetic beads the retained ssDNA fragments.

10. A method for genome-wide copy number profiling, comprising the steps of the method for low-pass whole genome sequencing according to any of the preceding claims,
- analysing the sequencing read density across different regions of the genome,
- determining a copy-number value for the regions of the genome by comparing the number of reads in that region with respect to the number of reads expected in the same for a reference genome.

11. A method for genome-wide copy number profiling according to claim 10, wherein the massively parallel sequencing library is prepared by using:
a) the primer of SEQ ID NO: 97 (Table 2) and one or more primers selected from the group consisting of SEQ ID NO:1 to SEQ ID NO: 96 (Table 2);
or
b) the primer of SEQ ID NO:194 (Table 2) and one or more primers selected from the group consisting of SEQ ID NO:98 to SEQ ID NO:193 (Table 2);
or
c) at least one primer selected from the group consisting of SEQ ID NO:195 to SEQ ID NO:202 (Table 4), and at least one primer selected from the group consisting of SEQ ID NO:203 to SEQ ID NO:214 (Table 4);
or
d) at least one primer selected from the group consisting of SEQ ID NO:215 to SEQ ID NO:222 (Table 6), and at least one primer selected from the group consisting of SEQ ID NO:223 to SEQ ID NO:234 (Table 6);
or
e) at least one primer selected from the group consisting of SEQ ID NO:235 to SEQ ID NO:242 (Table 7), and at least one primer selected from the group consisting of SEQ ID NO:243 to SEQ ID NO:254 (Table 7),
or
f) at least one primer selected from the group consisting of SEQ ID NO:259 to SEQ ID NO:266 (Table 8), and at least one primer selected from the group consisting of SEQ ID NO:267 to SEQ ID NO:278 (Table 8).
- analysing the sequencing read density across different regions of the genome,
- determining a copy-number value for the regions of the genome by comparing the number of reads in that region with respect to the number of reads expected in the same for a reference genome.

12. A method for genome-wide copy number profiling according to claim 10, wherein the massively parallel sequencing library is prepared by using - at least one primer selected from the group consisting of SEQ ID NO:235 to SEQ ID NO:242 (Table 7); at least one primer selected from the group consisting of SEQ ID NO:243 to SEQ ID NO:254 (Table 7); a custom sequencing primer of SEQ ID NO:255; and a primer of SEQ ID NO:256 or SEQ ID NO:258;
or
- at least one primer selected from the group consisting of SEQ ID NO:259 to SEQ ID NO:266 (Table 8); at least one primer selected from the group consisting of SEQ ID NO:267 to SEQ ID NO:278 (Table 8); and primers of SEQ ID NO:279 and SEQ ID NO:280;
designed to carry out an optimum single read sequencing process.

13. A method for genome-wide copy number profiling according to claim 12, wherein the massively parallel sequencing library is prepared by additionally using a primer selected from SEQ ID NO:257 (Table 7) and SEQ ID NO:281 (Table 8) designed to carry out an optimum Paired-End sequencing process in a selected sequencing platform.

## Patentansprüche

1. Verfahren zur Tiefpass-Gesamtgenomsequenzierung, umfassend die Schritte:
- Bereitstellen einer Vielzahl von mit Barcodes versehenen, massiv-parallelen Sequenzierungsbibliotheken, die durch ein Verfahren zur Erzeugung einer massiv-parallelen Sequenzierungsbibliothek erhalten werden, das die folgenden Schritte umfasst:
- Bereitstellen einer primären WGA-DNA-Bibliothek (pWGAlib), die Fragmente enthält, die aus einem bekannten 5'-Sequenzabschnitt (SSS), einem mittleren Sequenzabschnitt (MSS) und einem bekannten 3'-Sequenzabschnitt (3SS), der umgekehrt komplementär zu dem bekannten 5'-Sequenzabschnitt ist, bestehen, wobei der bekannte 5'-Sequenzabschnitt (SSS) aus einem universellen Sequenzadapter der DRS-WGA-Bibliothek oder einem universellen Sequenzadapter der MALBAC-Bibliothek besteht, und wobei der mittlere Sequenzabschnitt (MSS) aus mindestens einem Insertionsabschnitt (IS), der einer DNA-Sequenz der ursprünglichen, nicht amplifizierten DNA vor der WGA entspricht, und gegebenenfalls einem flankierenden 5'-Zwischenabschnitt (F5) und/oder einem flankierenden 3'-Zwischenabschnitt (F3) besteht,
- erneutes Amplifizieren der primären WGA-DNA-Bibliothek unter Verwendung mindestens eines ersten Primers (1PR) und mindestens eines zweiten Primers (2PR), wobei
der mindestens eine erste Primer (1PR) einen ersten Primer-5'-Abschnitt (1PRSS) und einen ersten Primer-3'-Abschnitt (1PR3S) umfasst, wobei der erste Primer-5'-Abschnitt (1PRSS) mindestens einen ersten Sequenzierungsadapter (1PR5SA) und mindestens einen ersten Sequenzierungsbarcode (1PR5BC) in 3'-Position des mindestens einen ersten Sequenzierungsadapters (1PR5SA) und in 5'-Position des ersten Primer-3'-Abschnitts (1PR3 S) umfasst, und wobei der erste Primer-3'-Abschnitt (1PR3S) entweder mit dem bekannten 5'-Sequenzabschnitt (SSS) oder dem bekannten 3'-Sequenzabschnitt (3SS) hybridisiert;
der mindestens eine zweite Primer (2PR) einen zweiten Primer-5'-Abschnitt (2PRSS) und einen zweiten Primer-3'-Abschnitt (2PR3S) umfasst, wobei der zweite Primer-5'-Abschnitt (2PRSS) mindestens einen zweiten Sequenzierungsadapter (2PRSSA) umfasst, der sich von dem mindestens einen ersten Sequenzierungsadapter (1PR5SA) unterscheidet, und wobei der zweite Primer-3'-Abschnitt (2PR3S) entweder mit dem bekannten 5'-Sequenzabschnitt (SSS) oder dem bekannten 3'-Sequenzabschnitt (3SS) hybridisiert
- Zusammenführen von Proben, die unter Verwendung verschiedener Sequenzierungsbarcodes (BC) erhalten wurden; und
- Sequenzieren der zusammengeführten Bibliothek.

2. Verfahren nach Anspruch 1, wobei der zweite Primer (2PR) ferner mindestens einen zweiten Sequenzierungsbarcode (2PRSBC) in der 3'-Position des mindestens einen zweiten Sequenzierungsadapters (2PRSSA) und in der 5'-Position des zweiten Primer-3'-Abschnitts (2PR3S) umfasst.

3. Verfahren nach Anspruch 1 oder 2, wobei der universelle Sequenzadapter der DRS-WGA-Bibliothek SEQ ID NO:282 ist und der universelle Sequenzadapter der MALBAC-Bibliothek SEQ ID NO:283 (MALBAC) ist.

4. Verfahren zur Tiefpass-Gesamtgenomsequenzierung nach einem der Ansprüche 1 bis 3, wobei der Schritt des Zusammenführens von Proben unter Verwendung verschiedener Sequenzierungsbarcodes (BC) ferner die Schritte umfasst:
- Quantifizieren der DNA in jeder der mit Barcodes versehenen, massiv-parallelen Sequenzierungsbibliotheken,
- Normalisieren der Menge der mit Barcodes versehenen, massiv-parallelen Sequenzierungsbibliotheken.

5. Verfahren zur Tiefpass-Gesamtgenomsequenzierung nach Anspruch 4, wobei der Schritt des Zusammenführens von Proben unter Verwendung verschiedener Sequenzierungsbarcodes (BC) ferner den Schritt des Auswählens von DNA-Fragmenten mit mindestens einem ausgewählten Bereich von Basenpaaren umfasst, wobei der Bereich von Basenpaaren auf 650 bp oder 400 bp oder 200 bp oder 150 bp oder 100 bp oder 50 bp konzentriert ist.

6. Verfahren zur Tiefpass-Gesamtgenomsequenzierung nach Anspruch 5, das ferner den Schritt des Auswählens von DNA-Fragmenten umfasst, die den ersten Sequenzierungsadapter und die zweiten Sequenzierungsadapter umfassen.

7. Verfahren zur Tiefpass-Gesamtgenomsequenzierung nach Anspruch 6, wobei der Schritt des Auswählens von DNA-Fragmenten, die den ersten Sequenzierungsadapter und die zweiten Sequenzierungsadapter umfassen, durch Inkontaktbringen der massiv-parallelen Sequenzierungsbibliothek mit paramagnetischen Beads, die mit einer Streptavidin-Beschichtung funktionalisiert sind, durchgeführt wird, und
wobei einer von dem mindestens einen ersten Primer (1PR) und dem mindestens einen zweiten Primer (2PR) am 5'-Ende biotinyliert ist und ausgewählte Fragmente durch Eluieren der nicht-biotinylierten ssDNA-Fragmente von den Beads erhalten werden.

8. Verfahren zur Tiefpass-Gesamtgenomsequenzierung nach Anspruch 7, wobei der mindestens eine zweite Primer am 5'-Ende biotinyliert ist.

9. Verfahren zur Tiefpass-Gesamtgenomsequenzierung nach Anspruch 7 oder 8, das ferner die weiteren Schritte umfasst:
- Inkubieren der erneut amplifizierten WGA-dsDNA-Bibliothek mit den funktionalisierten paramagnetischen Beads unter bestimmten Bedingungen, die eine kovalente Bindung zwischen Biotin und Streptavidin in den funktionalisierten paramagnetischen Kügelchen bewirken;
- Auswaschen der ungebundenen, nicht-biotinylierten dsDNA-Fragmente;
- Eluieren der zurückgehaltenen ssDNA-Fragmente von den funktionalisierten paramagnetischen Beads.

10. Verfahren zur genomweiten Erstellung von Kopienzahlprofilen, umfassend die Schritte des Verfahrens zur Tiefpass-Gesamtgenomsequenzierung nach einem der vorhergehenden Ansprüche,
- Analysieren der Sequenzierungslesedichte in verschiedenen Regionen des Genoms,
- Bestimmen eines Kopienzahlwertes für die Regionen des Genoms durch Vergleichen der Anzahl von Lesevorgängen in dieser Region mit der Anzahl von Lesevorgängen, die in derselben für ein Referenzgenom erwartet werden.

11. Verfahren zur genomweiten Erstellung von Kopienzahlprofilen nach Anspruch 10, wobei die massiv-parallele Sequenzierungsbibliothek hergestellt wird durch Verwendung von:
a) dem Primer der SEQ ID NO:97 (Tabelle 2) und einem oder mehreren Primer(n), ausgewählt aus der Gruppe bestehend aus SEQ ID NO:1 bis SEQ ID NO:96 (Tabelle 2);
oder
b) dem Primer der SEQ ID NO:194 (Tabelle 2) und einem oder mehreren Primer(n), ausgewählt aus der Gruppe bestehend aus SEQ ID NO:98 bis SEQ ID NO:193 (Tabelle 2);
oder
c) mindestens einem Primer, ausgewählt aus der Gruppe bestehend aus SEQ ID NO:195 bis SEQ ID NO:202 (Tabelle 4), und mindestens einem Primer, ausgewählt aus der Gruppe bestehend aus SEQ ID NO:203 bis SEQ ID NO:214 (Tabelle 4);
oder
d) mindestens einem Primer, ausgewählt aus der Gruppe bestehend aus SEQ ID NO:215 bis SEQ ID NO:222 (Tabelle 6), und mindestens einem Primer, ausgewählt aus der Gruppe bestehend aus SEQ ID NO:223 bis SEQ ID NO:234 (Tabelle 6),
oder
e) mindestens einem Primer, ausgewählt aus der Gruppe bestehend aus SEQ ID NO:235 bis SEQ ID NO:242 (Tabelle 7), und mindestens einem Primer, ausgewählt aus der Gruppe bestehend aus SEQ ID NO:243 bis SEQ ID NO:254 (Tabelle 7),
oder
f) mindestens einem Primer, ausgewählt aus der Gruppe bestehend aus SEQ ID NO:259 bis SEQ ID NO:266 (Tabelle 8), und mindestens einen Primer, ausgewählt aus der Gruppe bestehend aus SEQ ID NO:267 bis SEQ ID NO:278 (Tabelle 8).
- Analysieren der Sequenzierungslesedichte in verschiedenen Regionen des Genoms,
- Bestimmen eines Kopienzahlwertes für die Regionen des Genoms durch Vergleichen der Anzahl von Lesevorgängen in dieser Region mit der Anzahl von Lesevorgängen, die in derselben für ein Referenzgenom erwartet werden.

12. Verfahren zur genomweiten Erstellung von Kopienzahlprofilen nach Anspruch 10, wobei die massiv-parallele Sequenzierungsbibliothek unter Verwendung von
- mindestens einem Primer, ausgewählt aus der Gruppe bestehend aus SEQ ID NO:235 bis SEQ ID NO:242 (Tabelle 7); mindestens einem Primer, ausgewählt aus der Gruppe bestehend aus SEQ ID NO:243 bis SEQ ID NO:254 (Tabelle 7); einem benutzerdefinierten Sequenzierungsprimer der SEQ ID NO:255; und einem Primer der SEQ ID NO:256 oder SEQ ID NO:258;
oder
- mindestens einem Primer, ausgewählt aus der Gruppe bestehend aus SEQ ID NO:259 bis SEQ ID NO:266 (Tabelle 8); mindestens einem Primer, ausgewählt aus der Gruppe bestehend aus SEQ ID NO:267 bis SEQ ID NO:278 (Tabelle 8); und den Primern der SEQ ID NO:279 und SEQ ID NO:280,
dafür ausgelegt, ein optimales Single-Read-Sequenzierungsverfahren durchzuführen, hergestellt wird.

13. Verfahren zur genomweiten Erstellung von Kopienzahlprofilen nach Anspruch 12, wobei die massiv-parallele Sequenzierungsbibliothek hergestellt wird, indem zusätzlich ein Primer, der aus SEQ ID NO:257 (Tabelle 7) und SEQ ID NO:281 (Tabelle 8) ausgewählt ist, verwendet wird, dafür ausgelegt, ein optimales Paired-End-Sequenzierungsverfahren in einer ausgewählten Sequenzierungsplattform durchzuführen.

## Revendications

1. Procédé de séquençage passe-bas de génome entier, comprenant les étapes de :
- fourniture d'une pluralité de bibliothèques de séquençage massivement parallèles dotées de codes à barres, obtenues par un procédé de génération d'une bibliothèque de séquençage massivement parallèle comprenant les étapes de :
- fourniture d'une bibliothèque d'ADN WGA (amplification du génome entier) primaires (pWGAlib) incluant des fragments consistant en une section de séquence en 5' connue (5SS), une section de séquence centrale (MSS), et une section de séquence en 3' connue (3SS) inverse complémentaire de la section de séquence en 5' connue, la section de séquence en 5' connue (5SS) consistant en un adaptateur de séquence universel de bibliothèque DRS-WGA ou d'un adaptateur de séquence universel de bibliothèque MALBAC, et la section de séquence centrale (MSS) consistant en au moins une section d'insertion (IS), correspondant à une séquence d'ADN de l'ADN non amplifié original avant WGA, et éventuellement une section intermédiaire en 5' flanquante (F5) et/ou une section intermédiaire en 3' flanquante (F3) ;
- réamplification de la bibliothèque d'ADN WGA utilisant au moins une première amorce (1PR) et au moins une deuxième amorce (2PR) ;
dans lequel
l'au moins une première amorce (1PR) comprend une première section en 5' d'amorce (1PR5S) et une première section en 3' d'amorce (1PR3S), la première section en 5' d'amorce (1PR5S) comprenant au moins un premier adaptateur de séquençage (1PR5SA) et au moins un premier code à barres de séquençage (1PR5BC) dans une position en 3' de l'au moins un premier adaptateur de séquençage (1PR5SA) et dans une position en 5' de la première section en 3' d'amorce (1PR3S), et la première section en 3' d'amorce (1PR3S) s'hybridant soit à la section de séquence en 5' connue (5SS) soit à la section de séquence en 3' connue (3SS) ;
l'au moins une deuxième amorce (2PR) comprend une deuxième section en 5' d'amorce (2PR5S) et une deuxième section en 3' d'amorce (2PR3S), la deuxième section en 5' d'amorce (2PR5S) comprenant au moins un deuxième adaptateur de séquençage (2PR5SA) différent de l'au moins un premier adaptateur de séquençage (1PR5SA), et la deuxième section en 3' d'amorce (2PR3S) s'hybridant soit à la section de séquence en 5' connue (5SS) soit à la section de séquence en 3' connue (3SS) ;
- regroupement d'échantillons obtenus utilisant différents codes à barres de séquençage (BC) ; et
- séquençage de la bibliothèque regroupée.

2. Procédé selon la revendication 1, dans lequel la deuxième amorce (2PR) comprend en outre au moins un deuxième code à barres de séquençage (2PR5BC), en position en 3' de l'au moins un deuxième adaptateur de séquençage (2PR5SA) et en position en 5' de la deuxième section en 3' d'amorce (2PR3S).

3. Procédé selon la revendication 1 ou 2, dans lequel l'adaptateur de séquence universel de bibliothèque DRS-WGA est la SEQ ID NO : 282 et l'adaptateur de séquence universel de bibliothèque MALBAC est la SEQ ID NO : 283 (MALBAC).

4. Procédé de séquençage passe-bas de génome entier selon l'une quelconque des revendications 1 à 3, dans lequel l'étape de regroupement d'échantillons utilisant différents codes à barres de séquençage (BC) comprend en outre les étapes de :
- quantification de l'ADN dans chacune des bibliothèques de séquençage massivement parallèles dotées de codes à barres ;
- normalisation de la quantité de bibliothèques de séquençage massivement parallèles dotées de codes à barres.

5. Procédé de séquençage passe-bas de génome entier selon la revendication 4, dans lequel l'étape de regroupement d'échantillons utilisant différents codes à barres de séquençage (BC) comprend en outre l'étape de sélection de fragments d'ADN ayant au moins une plage sélectionnée de paires de bases, dans lequel la plage de paires de bases est centrée sur 650 pb, ou 400 pb, ou 200 pb, ou 150 pb, ou 100 pb, ou 50 pb.

6. Procédé de séquençage passe-bas de génome entier selon la revendication 5, comprenant en outre l'étape de sélection de fragments d'ADN comprenant le premier adaptateur de séquençage et les deuxièmes adaptateurs de séquençage.

7. Procédé de séquençage passe-bas de génome entier selon la revendication 6, dans lequel l'étape de sélection de fragments d'ADN comprenant le premier adaptateur de séquençage et les deuxièmes adaptateurs de séquençage est effectuée par mise en contact de la bibliothèque de séquençage massivement parallèle avec des perles paramagnétiques fonctionnalisées avec un revêtement de streptavidine, et
dans lequel l'une parmi l'au moins une première amorce (1PR) et l'au moins une deuxième amorce (2PR) est biotinylée à l'extrémité 5', et des fragments sélectionnés sont obtenus par élution de fragments d'ADNss à partir des perles non biotinylées.

8. Procédé de séquençage passe-bas de génome entier selon la revendication 7, dans lequel l'au moins une deuxième amorce est biotinylée à l'extrémité 5'.

9. Procédé de séquençage passe-bas de génome entier selon la revendication 7 ou 8, comprenant les étapes supplémentaires de :
- incubation de la bibliothèque d'ADNds WGA réamplifiée avec les perles paramagnétiques fonctionnalisées dans des conditions précisées provoquant ainsi une liaison covalente entre la biotine et la streptavidine assignées dans les perles paramagnétiques fonctionnalisées ;
- élimination par lavage de fragments d'ADNds non biotinylés non liés ;
- élution des fragments d'ADNss retenus à partir des perles paramagnétiques fonctionnalisées.

10. Procédé de profilage du nombre de copies sur tout un génome, comprenant les étapes du procédé de séquençage passe-bas de génome entier selon l'une quelconque des revendications précédentes,
- l'analyse de la densité de lectures de séquençage parmi différentes régions du génome,
- la détermination d'une valeur de nombre de copies pour les régions du génome par comparaison du nombre de lectures dans cette région avec le nombre de lectures prévu dans celle-ci pour un génome de référence.

11. Procédé de profilage du nombre de copies sur tout un génome selon la revendication 10, dans lequel la bibliothèque de séquençage massivement parallèle est préparée par utilisation de :
a) l'amorce de la SEQ ID NO : 97 (Tableau 2) et une ou plusieurs amorces choisies dans le groupe consistant en les SEQ ID NO : 1 à 96 (Tableau 2) ; ou
b) l'amorce de la SEQ ID NO : 194 (Tableau 2) et une ou plusieurs choisies dans le groupe consistant en les SEQ ID NO : 98 à 193 (Tableau 2) ; ou
c) au moins une amorce choisie dans le groupe constitué par les SEQ ID NO : 195 à 202 (Tableau 4), et au moins une amorce choisie dans le groupe constitué par les SEQ ID NO : 203 à 214 (Tableau 4) ; ou
d) au moins une amorce choisie dans le groupe constitué par les SEQ ID NO : 215 à 222 (Tableau 6), et au moins une amorce choisie dans le groupe constitué par les SEQ ID NO : 223 à 234 (Tableau 6) ; ou
e) au moins une amorce choisie dans le groupe constitué par les SEQ ID NO : 235 à 242 (Tableau 7), et au moins une amorce choisie dans le groupe constitué par les SEQ ID NO : 243 à 254 (Tableau 7), ou
f) au moins une amorce choisie dans le groupe constitué par les SEQ ID NO : 259 à 266 (Tableau 8), et au moins une amorce choisie dans le groupe constitué par les SEQ ID NO : 267 à 278 (Tableau 8) ;
- l'analyse de la densité de lectures de séquençage parmi différentes régions du génome,
- la détermination d'une valeur de nombre de copies pour les régions du génome par comparaison du nombre de lectures dans cette région avec le nombre de lectures prévu dans la même pour un génome de référence.

12. Procédé de profilage du nombre de copies sur tout un génome selon la revendication 10, dans lequel la bibliothèque de séquençage massivement parallèle est préparée par utilisation de :
- au moins une amorce choisie dans le groupe constitué par les SEQ ID NO : 235 à 242 (tableau 7) ; d'au moins une amorce choisie dans le groupe constitué par les SEQ ID NO : 243 à 254 (tableau 7) ; d'une amorce de séquençage sur mesure de la SEQ ID NO : 255 ; et d'une amorce de la SEQ ID NO : 256 ou de la SEQ ID NO : 258 ; ou
- au moins une amorce choisie dans le groupe constitué par les SEQ ID NO : 259 à 266 (tableau 8) ; d'au moins une amorce choisie dans le groupe constitué par les SEQ ID NO : 267 à 278 (tableau 8) ; et d'amorces de la SEQ ID NO : 279 et de la SEQ ID NO : 280 ;
conçues pour mettre en œuvre un processus optimal de séquençage de lectures uniques.

13. Procédé de profilage du nombre de copies sur tout un génome selon la revendication 12, dans lequel la bibliothèque de séquençage massivement parallèle est préparée par utilisation de plus d'une amorce choisie parmi la SEQ ID NO : 257 (Tableau 7) et la SEQ ID NO : 281 (Tableau 8), conçue pour mettre en œuvre un processus optimal de séquençage d'extrémités appariées dans une plateforme de séquençage sélectionnée.
